# FASCICULE DE BREVET EUROPEEN

(11) **EP 2 931 363 B1**
(45) Date de publication et mention de la délivrance du brevet: **10.01.2018**
(21) Numéro de dépôt: 13805854.0
(22) Date de dépôt: 12.12.2013
(51) Int. Cl.: A61N 1/375, H05K 5/00

(54) **BOITIER HERMETIQUE POUR L'ENCAPSULATION D'UN DISPOSITIF MEDICAL IMPLANTABLE**
HERMETISCHES GEHÄUSE ZUR EINKAPSELUNG EINER IMPLANTIERBAREN MEDIZINISCHEN VORRICHTUNG
HERMETIC HOUSING FOR ENCAPSULATING AN IMPLANTABLE MEDICAL DEVICE

(30) Priorité: 14.12.2012 FR 1262075
(43) Date de publication de la demande: 21.10.2015
(73) Titulaire: Commissariat à l'Énergie Atomique et aux Énergies Alternatives, 75015 Paris (FR)
(72) Inventeur: PERRAUD, Simon, 83150 Bandol (FR); EMIEUX, Fabrice, 38340 Voreppe (FR); KARST, Nicolas, 57600 Folkling (FR); LE COADOU, Cécile, 38000 Grenoble (FR)
(74) Mandataire: Gevers & Orès
(86) Numéro de dépôt international: PCT/EP2013/076353
(87) Numéro de publication internationale: WO 2014/090937

(56) Documents cités:
- FR-A1- 2 972 852
- FR-A1- 2 974 745
- US-A- 5 750 926
- US-A- 5 913 881
- US-A- 6 010 803
- US-A1- 2002 138 114
- US-A1- 2004 176 817
- US-A1- 2008 294 207
- US-A1- 2009 266 573
- US-A1- 2010 114 225
- US-A1- 2010 274 309
- US-A1- 2012 193 117

## Description

L'invention porte sur un boitier hermétique ou étanche, de préférence ultrafin et/ou conformable, pouvant notamment convenir à l'encapsulation d'un dispositif et plus particulièrement, mais pas exclusivement, d'un dispositif médical implantable. L'invention porte également sur un procédé d'encapsulation d'un dispositif, et notamment d'un dispositif médical implantable.

Les dispositifs médicaux implantables tels que les stimulateurs cardiaques, les défibrillateurs cardiaques, les moniteurs cardiaques, les neurostimulateurs, les pompes, les capteurs biomédicaux, etc. sont généralement encapsulés dans un boîtier métallique biocompatible, typiquement en titane, qui est clos hermétiquement par soudage au faisceau laser. Ce type d'encapsulation assure une excellente herméticité, afin d'une part de protéger les composants encapsulés contre les attaques par les tissus ou fluides biologiques, et d'autre part de protéger le corps du patient contre les éventuels éléments encapsulés qui soient toxiques ou non biocompatibles. Des traversées verre-métal ou céramique-métal sont aménagées au niveau des parois du boîtier, afin de permettre la sortie d'une ou plusieurs électrodes tout en conservant une parfaite herméticité. De tels boîtiers métalliques sont bien adaptés pour encapsuler des dispositifs médicaux implantables se présentant sous la forme d'objets volumineux (d'épaisseur typique comprise entre 5 et 20 mm) et mécaniquement rigides.

Une solution alternative d'encapsulation de dispositifs médicaux implantables consiste à assembler un couvercle (c'est-à-dire une pièce dont la forme ressemble à celle d'un chapeau) sur un substrat (c'est-à-dire une pièce plane), comme décrits par exemple dans le document US 5,750,926 et dans l'article de A. Vanhoestenberghe et al.,« Hermetic Encapsulation of an Implantable Vision Prosthesis - Combining Implant Fabrication Philosophies » (Proceedings of IFESS 2008, 21- 25 septembre 2008, Fribourg). Dans le document US 5,750,926, le boîtier consiste en un couvercle métallique fixé sur un substrat électriquement isolant ; l'herméticité entre le couvercle et le substrat peut être assurée en formant un premier joint hermétique entre un cadre métallique et le substrat, puis un second joint hermétique par soudage au faisceau laser entre le cadre métallique et le couvercle. Dans l'article précité de A. Vanhoestenberghe et al., le boîtier consiste en un couvercle en céramique fixé sur un substrat en céramique ; l'herméticité entre le couvercle et le substrat est assurée en formant un premier joint hermétique par brasage entre un cadre en titane et le substrat, un deuxième joint hermétique par brasage entre un autre cadre en titane et le couvercle, puis un troisième joint hermétique par soudage au faisceau laser entre les deux cadres en titane. Dans ces deux documents, la sortie hermétique d'une ou plusieurs électrodes est effectuée par des pistes métalliques et des traversées métalliques verticales.

Les méthodes d'encapsulation décrites par ces documents sont bien adaptées pour obtenir des dispositifs après encapsulation qui soient fins (d'épaisseur typique de quelques millimètres) et mécaniquement rigides, comme démontré expérimentalement dans l'article de Vanhoestenberghe et al. Il est également envisageable d'utiliser ce type d'encapsulation pour obtenir des dispositifs après encapsulation qui soient ultrafins (d'épaisseur inférieure à 3 mm), comme décrit dans le document précité US 5,750,926. Cependant, en pratique, il est techniquement difficile de réduire l'épaisseur du dispositif après encapsulation sous la barre des 3 mm. En particulier, l'utilisation de cadres métalliques ultrafins rend extrêmement délicate la formation du dernier joint hermétique par soudage au faisceau laser, car si les cadres sont trop fins alors l'étape de soudage au faisceau laser risque de détériorer thermiquement le ou les joints hermétiques préalablement formés. US2008/294207 divulgue un boîtier hermétique comprenant : un premier élément en forme de feuille, réalisé en métal ; un deuxième élément de dimensions et forme adaptés pour recouvrir ledit premier élément, également réalisé en métal ; un premier cadre métallique interposé entre ledit premier élément et ledit deuxième élément ; un premier joint hermétique entre ledit premier élément et ledit premier cadre métallique ; et un deuxième joint hermétique entre ledit deuxième élément et ledit premier cadre métallique.

Il n'existe donc pas dans l'art antérieur de solution totalement satisfaisante - c'est-à-dire simple et fiable - pour obtenir des dispositifs après encapsulation qui soient ultrafins, voire mécaniquement conformables. Or de tels dispositifs permettraient d'améliorer sensiblement le confort du patient, et d'envisager une implantation dans des zones du corps humain difficilement accessibles à des dispositifs conventionnels. Par exemple, un dispositif implantable de stimulation cérébrale se présentant sous la forme d'un objet ultrafin et mécaniquement conformable pourrait être implanté au niveau de la tête, sous le cuir chevelu, au lieu d'être implanté de manière conventionnelle au niveau du haut de la poitrine. Une telle implantation au plus près de la zone à stimuler permettrait d'éviter de recourir à de longues sondes-électrodes reliant la poitrine à la tête, de telles sondes-électrodes présentant des risques de rupture en particulier au niveau du cou. Pour ce type d'implantation, le dispositif de stimulation cérébrale devrait alors présenter une épaisseur inférieure à 1 mm, et pouvoir présenter un rayon de courbure de l'ordre de quelques centimètres.

L'invention vise à surmonter les inconvénients précités de l'art antérieur. Plus précisément, l'invention vise à procurer un boîtier hermétique et, si nécessaire, biocompatible, pouvant être réalisé aisément sous forme ultrafine et, le cas échéant, mécaniquement conformable. L'invention vise également à procurer un procédé simple et fiable pour l'encapsulation d'un dispositif, et notamment d'un dispositif médical implantable, ce procédé étant en particulier adapté à la réalisation d'un assemblage ultrafin et/ou conformable. Un dispositif, boîtier ou assemblage est considéré « ultrafin » lorsqu'il présente une épaisseur maximale inférieure ou égale à 3 mm, et de préférence inférieure ou égale à 1 mm, voire à 500 µm.

Ainsi, un objet de l'invention est un boîtier hermétique comprenant :
- un premier élément en forme de feuille, réalisé en au moins un matériau choisi parmi un métal, une céramique et un verre ;
- un deuxième élément de dimensions et forme adaptés pour recouvrir ledit premier élément, également réalisé en au moins un matériau choisi parmi un métal, une céramique et un verre ;
- un premier cadre métallique interposé entre ledit premier élément et ledit deuxième élément, comprenant une partie interne positionnée en partie ou en totalité à l'intérieur du périmètre dudit premier élément et une partie externe positionnée en totalité à l'extérieur dudit périmètre dudit premier élément ;

- un premier joint hermétique entre ledit premier élément et ladite partie interne dudit premier cadre métallique ; et
- un deuxième joint hermétique entre ledit deuxième élément et ladite partie externe dudit premier cadre métallique.

On entend par « élément en forme de feuille » ou simplement « feuille » un élément présentant une épaisseur non supérieure à un dixième, de préférence un cinquantième et manière encore préférée un centième, de sa plus petite dimension latérale.

Avantageusement, ledit premier joint hermétique peut être réalisé par une technique choisie parmi : le brasage, le soudage par diffusion à l'état solide et, lorsque ledit premier élément est au moins en partie en céramique, le cofrittage ; et ledit deuxième joint hermétique peut être réalisé par soudage.

Selon un premier mode de réalisation de l'invention, ledit deuxième élément peut comprendre :
- une feuille dite supérieure, réalisée en au moins un matériau choisi parmi un métal, une céramique et un verre ;
- un deuxième cadre métallique interposé entre ladite feuille supérieure et ledit premier cadre métallique, comprenant une partie interne positionnée en partie ou en totalité à l'intérieur du périmètre de ladite feuille supérieure et une partie externe positionnée en totalité à l'extérieur dudit périmètre de ladite feuille supérieure ; et
- un troisième joint hermétique entre ladite feuille supérieure et ladite partie interne dudit deuxième cadre métallique ;
ledit deuxième joint hermétique étant entre les parties externes desdits premier et deuxième cadre métallique.

Avantageusement, ledit troisième joint hermétique peut être réalisé par une technique choisie parmi : le brasage, le soudage par diffusion à l'état solide et, lorsque ladite feuille supérieure est au moins en partie en céramique, le cofrittage.

Selon un deuxième mode de réalisation de l'invention, ledit deuxième élément peut comprendre une feuille dite supérieure en métal, directement fixée à la partie externe dudit premier cadre métallique par ledit deuxième joint hermétique.

Aussi bien dans le premier que dans le deuxième mode de réalisation de l'invention, la partie externe dudit ou de chaque cadre métallique peut présenter, par rapport à la partie interne correspondante, une surépaisseur sur sa face opposée audit deuxième joint hermétique. Cela facilite la réalisation du deuxième joint hermétique par soudure, sans pour autant augmenter l'épaisseur totale du boîtier ou compromettre sa flexibilité, lorsqu'une conformabilité mécanique est souhaitée.

Le boîtier hermétique peut présenter une épaisseur inférieure ou égale à 3 mm, de préférence inférieure ou égale à 1 mm et de manière encore préférée inférieure ou égale à 500 µm, et être donc « ultrafin ».

Le boîtier peut comporter au moins un composant, notamment électronique ou électrique, monté sur ledit premier élément en forme de feuille, directement ou par l'intermédiaire d'une couche diélectrique, et contenu dans un espace délimité par ledit premier élément, ledit deuxième élément et ledit ou lesdits cadres métalliques. Avantageusement, ledit ou chaque composant peut être recouvert d'une couche en matériau polymère. Afin de permettre l'interconnexion du ou des composants électroniques contenus dans le boîtier avec des éléments extérieurs sans compromettre l'étanchéité de l'ensemble, le boîtier peut comprendre au moins une piste conductrice agencée sur ledit premier élément en forme de feuille, ladite ou chaque piste conductrice s'étendant sur les deux faces et sur la tranche dudit premier élément et étant recouverte d'un matériau isolant sauf au niveau de ses extrémités, formant plages de connexion.

Un tel boîtier hermétique peut être partiellement ou totalement enrobé d'une couche en matériau polymère.

Dans le cas d'une réalisation implantable dans un corps humain ou animal, le boîtier devra être réalisé totalement ou au moins partiellement (en particulier en ce qui concerne sa surface externe) en matériau ou matériaux biocompatible(s).

D'autres caractéristiques de l'invention sont présentées dans la description qui suit la définition des dessins annexés. Ces caractéristiques portent notamment sur la flexibilité du boîtier, la connexion de pistes métalliques/électrodes hermétiques ou encore la conformation des feuilles inférieure ou supérieure, en particulier pour définir une cavité dans le boîtier.

Un autre objet de l'invention est un dispositif médical implantable comprenant un boîtier hermétique tel que décrit ci-dessus.

Encore un autre objet de l'invention est un procédé d'encapsulation d'un dispositif (en particulier d'un dispositif implantable, encore plus particulièrement d'un dispositif médical implantable), comprenant les étapes consistant à :
- monter au moins un composant dudit dispositif à encapsuler sur un premier élément en forme de feuille, réalisé en au moins un matériau choisi parmi un métal, une céramique et un verre ;
- superposer audit premier élément en forme de feuille un premier cadre métallique comprenant une partie interne positionnée en partie ou en totalité à l'intérieur du périmètre dudit premier élément et une partie externe positionnée en totalité à l'extérieur dudit périmètre dudit premier élément ;
- former un premier joint hermétique entre ledit premier élément et ladite partie interne dudit premier cadre métallique ;
- superposer audit premier cadre métallique un deuxième élément de dimensions et forme adaptés pour recouvrir ledit premier élément, également réalisé en au moins un matériau choisi parmi un métal, une céramique et un verre ; et
- former un deuxième joint hermétique entre ledit deuxième élément et ladite partie externe dudit premier cadre métallique.

Avantageusement, l'étape de formation dudit premier joint hermétique peut être réalisée par une technique choisie parmi : le brasage, le soudage par diffusion à l'état solide et, lorsque ledit deuxième élément est au moins en partie en céramique, le cofrittage ; et l'étape de formation dudit deuxième joint hermétique peut être réalisée par soudage.

Selon un premier mode de réalisation de ce procédé, ledit deuxième élément peut comprendre :
- une feuille dite supérieure, réalisée en au moins un matériau choisi parmi un métal, une céramique et un verre ;
- un deuxième cadre métallique interposé entre ladite feuille supérieure et ledit premier cadre métallique et comprenant une partie interne positionnée en partie ou en totalité à l'intérieur du périmètre de ladite feuille supérieure et une partie externe positionnée en totalité à l'extérieur dudit périmètre de ladite feuille supérieure ;

le procédé comprenant également une étape de formation d'un troisième joint hermétique entre ladite feuille supérieure et ladite partie interne dudit deuxième cadre métallique, ladite étape étant réalisée avant la formation dudit deuxième joint hermétique entre les parties externes desdits premier et deuxième cadre métallique.

Avantageusement, l'étape de formation dudit troisième joint hermétique peut être réalisée par une technique choisie parmi : le brasage, le soudage par diffusion à l'état solide et, lorsque ladite feuille supérieure est au moins en partie en céramique, le cofrittage.

Selon un deuxième mode de réalisation de ce procédé, ledit deuxième élément peut comprendre une feuille dite supérieure en métal, ledit deuxième joint hermétique étant alors formé directement entre ladite feuille supérieure et la partie externe dudit premier cadre métallique.

D'autres caractéristiques, détails et avantages de l'invention ressortiront à la lecture de la description faite en référence aux dessins annexés donnés à titre d'exemple et dans lesquels :
- la figure 1 (1A - 1D) représente schématiquement les différentes pièces d'un boîtier selon le premier mode de réalisation de l'invention avant leur assemblage ;
- la figure 2 représente un boîtier selon le premier mode de réalisation de l'invention une fois assemblé ; en particulier la figure 2A correspond à une vue de dessus et la figure 2B à une vue en coupe ;
- la figure 3 (3A, 3B) illustre la réalisation par soudage laser du deuxième joint hermétique d'un boîtier selon le premier mode de réalisation de l'invention;
- la figure 4 illustre la « pré-encapsulation » de composants électroniques à l'intérieur d'un boîtier selon le premier mode de réalisation de l'invention (4A, 4B), la « post-encapsulation » du boîtier lui-même (4C) ;
- la figure 5 (5A - 5D) illustre la réalisation d'une sortie hermétique pour une ou plusieurs pistes conductrices ;
- la figure 6 (6A - 6C) représente schématiquement les différentes pièces d'un boîtier selon le deuxième mode de réalisation de l'invention avant leur assemblage ;
- la figure 7 représente un boîtier selon le deuxième mode de réalisation de l'invention une fois assemblé ; en particulier la figure 7A correspond à une vue de dessus et la figure 7B à une vue en coupe ;
- la figure 8 (8A, 8B) illustre la réalisation par soudure laser du deuxième joint hermétique d'un boîtier selon le deuxième mode de réalisation de l'invention;
- la figure 9 illustre la « pré-encapsulation » de composants électroniques à l'intérieur d'un boîtier selon le deuxième mode de réalisation de l'invention, et la « post-encapsulation » du boîtier lui-même ; et
- les figures 10 (10A, 10B, 10C) et 11 représentent deux variantes de boîtiers selon le deuxième mode de réalisation de l'invention ;
- la figure 12 (12A, 12B, 12C) illustre des moyens pour améliorer la flexibilité mécanique d'un boîtier ou d'un ensemble de boîtiers selon l'invention selon plusieurs axes (figure 12A = référence);
- la figure 13 (13A, 13B, 13C) illustre la sortie d'une ou plusieurs pistes électriques formant électrodes ou plages de connexion d'un boîtier conforme à l'invention, la feuille inférieure dudit boitier est en céramique;
- la figure 14 (14A, 14B, 14C) illustre un autre type de sortie d'une ou plusieurs pistes électriques formant électrodes ou plages de connexion d'un boîtier conforme à l'invention, dans le cas où la feuille inférieure dudit boitier est en céramique;
- la figure 15 (15A, 15B, 15C) illustre encore un autre type de sortie d'une plusieurs pistes électriques formant électrodes ou plages de connexion d'un boîtier conforme à l'invention, dans le cas où la feuille inférieure dudit boitier est en métal.

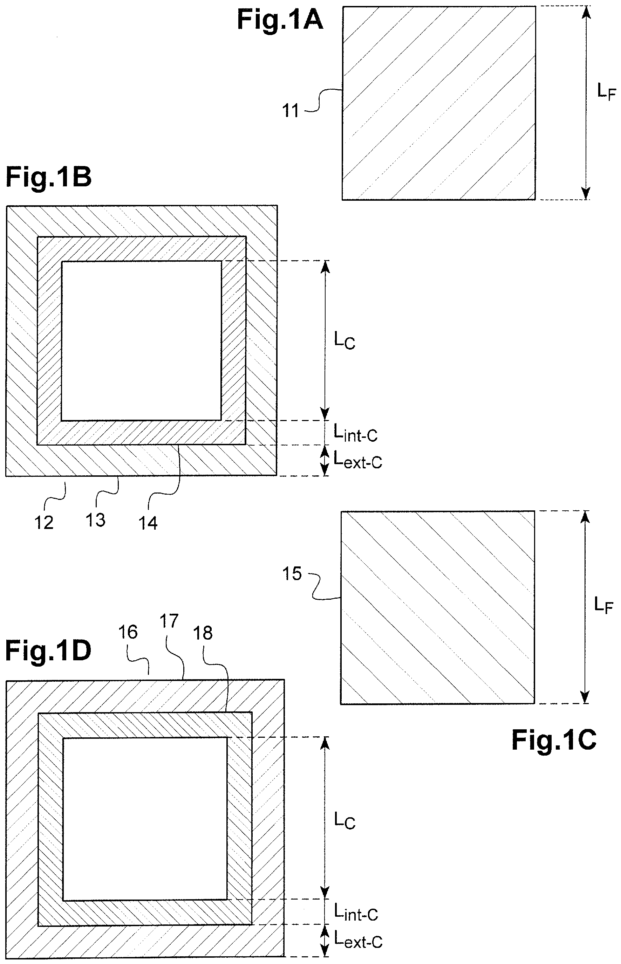

Les figures 1 à 5 illustrent un boîtier selon un premier mode de réalisation de l'invention et les différentes étapes de son procédé de fabrication. En particulier, la figure 1 (vue de dessus) représente schématiquement les différentes pièces du boîtier, avant leur assemblage. Ces pièces sont un premier élément en forme de feuille, ou « feuille inférieure » 11, servant de support au dispositif à encapsuler, un premier cadre métallique 12, ou « cadre métallique inférieur » et un deuxième élément servant de couvercle, constitué à son tour par un deuxième élément, également en forme de feuille et dit « feuille supérieure » 15 et par un deuxième cadre métallique, ou « cadre métallique supérieur » 16.

La figure 1A représente la feuille inférieure 11 qui, dans le mode de réalisation exemplaire considéré ici, est une pièce de forme carrée, de côté noté L_{F}, avantageusement compris entre 5 mm et 10 cm. D'autres formes sont envisageables, en particulier une forme circulaire. L'épaisseur de la feuille inférieure 11, notée e_{FI}, est avantageusement inférieure à 500µm, de préférence inférieure à 50 µm. La feuille inférieure 11 est avantageusement une pièce en céramique (de préférence en zircone ZrO₂ stabilisée à l'oxyde d'yttrium Y₂O₃ à 3% molaire ou plus, ou en zircone ZrO₂ stabilisée à l'oxyde de cérium CeO₂), ou en métal (de préférence en titane ou en alliage de titane). La feuille inférieure 11 peut être également une pièce en verre. Un avantage de la céramique ou du verre par rapport au métal est sa meilleure transparence aux rayonnements électromagnétiques ou lumineux, ce qui peut être utile dans certaines applications. Un avantage de la zircone stabilisée par rapport au verre est la meilleure résistance mécanique (en particulier la meilleure ténacité). Un avantage du métal par rapport à la céramique et au verre est sa meilleure résistance mécanique.

La figure 1B représente le premier cadre métallique, ou cadre métallique inférieur 12, qui est une pièce de forme carrée (plus généralement, épousant la forme de la feuille inférieure), et présentant une ouverture centrale de forme carrée de côté noté L_{C}, avantageusement compris entre 5 mm et 10 cm. Le cadre métallique inférieur 12 comprend deux parties :
- une partie externe 13, de largeur notée L_{ext-C} (avantageusement comprise entre 500 µm et 1 cm), et d'épaisseur notée e_{ext-CI} (avantageusement inférieure à 500 µm, de préférence inférieure à 50 µm) ;
- une partie interne 14, de largeur notée L_{int-C} (avantageusement comprise entre 500 µm et 1 cm), et d'épaisseur notée e_{int-CI} inférieure ou égale à e_{ext-CI}.

La partie externe 13 et la partie interne 14 constituent, dans le cas considéré ici, une seule et unique pièce monolithique 12. Le cadre métallique inférieur 12 est avantageusement une pièce en titane ou en alliage de titane.

La figure 1C représente la feuille supérieure 15 qui, dans le mode de réalisation exemplaire considéré ici, est une pièce de forme carrée, de côté égal à L_{F}. L'épaisseur de la feuille supérieure 15, notée e_{FS}, est avantageusement inférieure à 500 µm, de préférence inférieure à 50 µm. La feuille supérieure 15 est avantageusement une pièce en céramique (de préférence en zircone ZrO₂ stabilisée à l'oxyde d'yttrium Y₂O₃ à 3% molaire ou plus, ou en zircone ZrO₂ stabilisée à l'oxyde de cérium CeO₂), ou en métal (de préférence en titane ou en alliage de titane). La feuille supérieure 15 peut être également une pièce en verre.

La figure 1D représente le deuxième cadre métallique, ou cadre métallique supérieur 16 qui, dans le mode de réalisation exemplaire considéré ici, est une pièce de forme carrée (plus généralement, épousant la forme de la feuille inférieure), présentant une ouverture centrale de forme carré de côté égal à L_{C}. Le cadre métallique inférieur 16 comprend lui aussi deux parties :
- une partie externe 17, de largeur égale à L_{ext-C}, et d'épaisseur notée e_{ext-CS} (avantageusement inférieure à 500 µm, de préférence inférieure à 50 µm);
- une partie interne 18, de largeur égale à L_{int-C}, et d'épaisseur notée e_{int-CS} inférieure ou égale à e_{ext-CS}.

La partie externe 17 et la partie interne 18 constituent, dans le cas considéré ici, une seule et unique pièce monolithique 16. Le cadre métallique supérieur 16 est avantageusement une pièce en titane ou en alliage de titane.

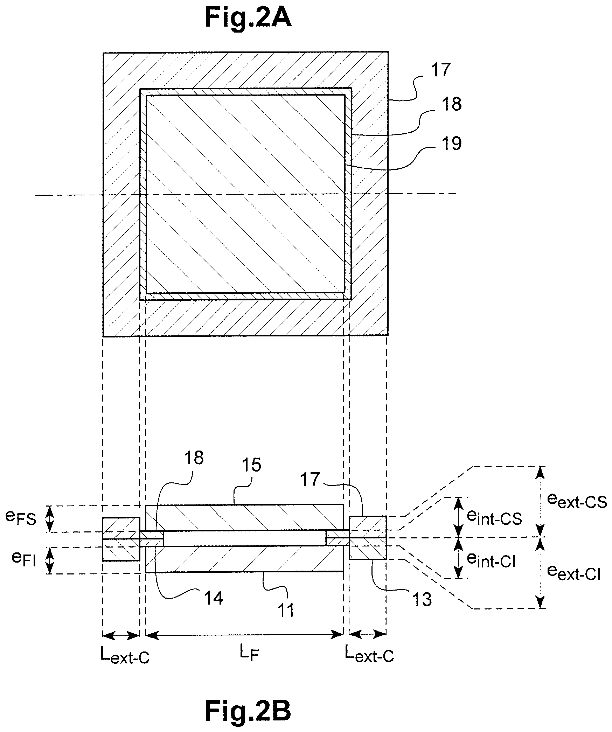

La figure 2 représente schématiquement le boîtier une fois les différentes pièces assemblées (feuille inférieure 11, cadre métallique inférieur 12, feuille supérieure 15, cadre métallique supérieur 16). La figure 2A est une vue de dessus, alors que la figure 2B est une vue en coupe selon l'axe représenté en pointillé sur la figure 2A. La partie externe 13 du cadre métallique inférieur 12 est positionnée en totalité à l'extérieur du périmètre délimité par la feuille inférieure 11, et la partie interne 14 du cadre métallique inférieur 12 est positionnée en partie à l'intérieur du périmètre délimité par la feuille inférieure 11. De même, la partie externe 17 du cadre métallique supérieur 16 est positionnée en totalité à l'extérieur du périmètre délimité par la feuille supérieure 15, et la partie interne 18 du cadre métallique supérieur 16 est positionnée en partie à l'intérieur du périmètre délimité par la feuille supérieure 15.

Un premier joint hermétique est formé entre la feuille inférieure 11 et la partie interne 14 du cadre métallique inférieur 12. Avantageusement, ce joint hermétique est formé par brasage. Dans le cas où la feuille inférieure 11 est en zircone et le cadre métallique inférieur 12 est en titane ou en alliage de titane, ce brasage peut être effectué dans un four sous vide secondaire, à une température de l'ordre de 1000°C, via un joint de brasure à base d'un alliage titane-nickel. On pourra par exemple utiliser un joint de brasure du type TiNi-50 (Ni 50% en masse) commercialisé par la société Wesgo. Alternativement, on pourra utiliser un joint de brasure contenant une quantité plus importante de nickel (c'est-à-dire supérieure à 50% en masse), le complément en titane étant directement apporté par le titane contenu dans le cadre métallique inférieur 12. Le joint de brasure peut être positionné légèrement en retrait (quelques mm) du bord de la feuille inférieure 11, afin notamment d'éviter des coulures du joint vers l'extérieur du périmètre délimité par la feuille 11 pendant le recuit de brasage. Avant d'effectuer le brasage, une couche mince métallique (titane, or, platine, etc.) peut être préalablement déposée (par dépôt physique en phase vapeur, dépôt chimique en phase vapeur, électrodépôt, enduction, impression, etc.) sur les bords de la feuille inférieure 11. Comme connu dans l'art antérieur (voir par exemple Jiang et al., « Technology advances and challenges in hermetic packaging for implantable médical devices », Implantable Neural Prostheses 2, Biological and Medical Physics, Biomedical Engineering 2010, pp 27-61), une telle couche mince métallique peut faciliter la formation du brasage d'une pièce métallique sur une pièce en céramique. Avant d'effectuer le brasage, une couche mince d'oxyde (alumine, silice, etc.) peut être préalablement déposée (par dépôt physique en phase vapeur, dépôt chimique en phase vapeur, enduction, impression, etc.) sur les bords ou la totalité de la feuille inférieure 11, afin de limiter le noircissement de la pièce en céramique lors du brasage (cet éventuel noircissement est dû à la diffusion de l'oxygène depuis la pièce en céramique vers le joint de brasure, conduisant à une pièce en céramique sous-stoechiométrique en oxygène).

En variante, le premier joint hermétique peut être réalisé par soudage par diffusion à l'état solide. En variante, lorsque la feuille inférieure est en céramique, le premier joint hermétique peut être réalisé par cofrittage de ladite feuille inférieure et dudit cadre métallique inférieur.

Un autre joint hermétique, dit « troisième joint hermétique », est également formé entre la feuille supérieure 15 et la partie interne 18 du cadre métallique supérieur 16. Avantageusement, ce joint hermétique est formé par brasage, par un procédé similaire à celui utilisé pour former le premier joint hermétique entre la feuille inférieure 11 et la partie interne 14 du cadre métallique inférieur 12.

Encore un autre joint hermétique, dit deuxième joint hermétique, est formé entre la partie externe 13 du cadre métallique inférieur 12 et la partie externe 17 du cadre métallique supérieur 16. Avantageusement, ce joint hermétique est formé par soudage au faisceau laser. Ce procédé se caractérise par un apport localisé de chaleur, le diamètre typique du faisceau laser pouvant être de l'ordre de quelques centaines de microns. D'autres procédés de soudage avec apport localisé de chaleur peuvent être utilisés, comme le soudage par faisceau d'électrons, ou le soudage par résistance (effet Joule).

Le fait que la partie externe 13 du cadre métallique inférieur 12 soit positionnée en totalité à l'extérieur du périmètre délimité par la feuille inférieure 11, et que la partie externe 17 du cadre métallique supérieur 16 soit positionnée en totalité à l'extérieur du périmètre délimité par la feuille supérieure 15 est particulièrement avantageux. En effet, une telle configuration géométrique permet de former un joint hermétique entre la partie externe 13 du cadre métallique inférieur 12 et la partie externe 17 du cadre métallique supérieur 16, sans pour autant dégrader thermiquement le joint hermétique entre la feuille inférieure 11 et la partie interne 14 du cadre métallique inférieur 12 (ce joint étant positionné à l'intérieur du périmètre délimité par la feuille inférieure 11) et/ou le joint hermétique entre la feuille supérieure 15 et la partie interne 18 du cadre métallique supérieur 16 (ce joint étant positionné à l'intérieur du périmètre délimité par la feuille supérieure 15).

Le fait que l'épaisseur e_{ext-CI} (respectivement e_{ext-CS}) de la partie externe 13 (respectivement 17) du cadre métallique inférieur 12 (respectivement du cadre métallique supérieur 16) soit supérieure ou égale à l'épaisseur e_{int-CI} (respectivement e_{int-CS}) de la partie interne 14 (respectivement 18) permet d'apporter une quantité de matière suffisante pour assurer la formation d'un joint hermétique entre la partie externe 13 et la partie externe 17, par un procédé tel que le soudage au faisceau laser. Plus particulièrement, la partie externe de chaque cadre peut présenter une surépaisseur orientée uniquement du côté opposé au deuxième joint hermétique formé entre les deux cadres. Cela permet de ne pas augmenter l'épaisseur totale de l'assemblage.

Le fait que l'épaisseur e_{int-CI} (respectivement e_{int-CS}) de la partie interne 14 (respectivement 18) du cadre métallique inférieur 12 (respectivement du cadre métallique supérieur 16) soit inférieure ou égale à l'épaisseur e_{ext-CI} (respectivement e_{ext-CS}) de la partie externe 13 (respectivement 17) permet d'assurer la finesse et la flexibilité mécanique du boîtier, et ce même au niveau des zones potentiellement les plus épaisses, c'est-à-dire des zones où se trouve un empilement feuille/cadre/cadre/feuille.

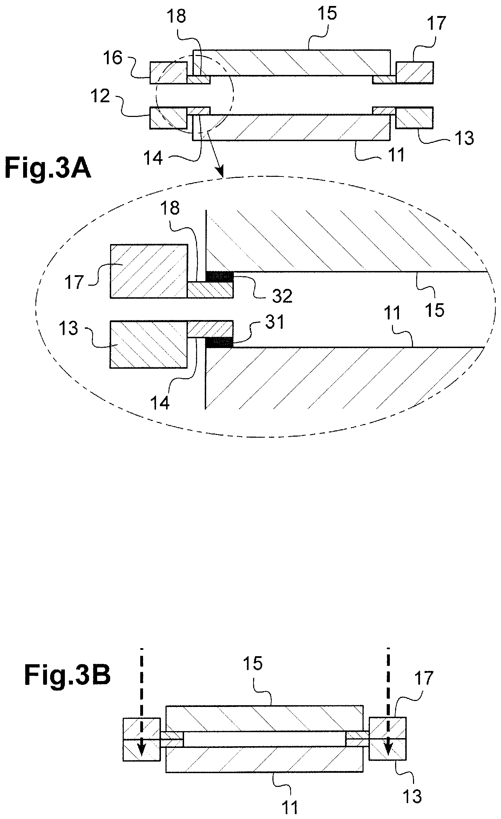

La figure 3 (vue en coupe) représente schématiquement le procédé de soudage au faisceau laser pour la formation du joint hermétique entre la partie externe 13 du cadre métallique inférieur 12 et la partie externe 17 du cadre métallique supérieur 16.

La figure 3A représente la partie inférieure du boîtier (qui comprend la feuille inférieure 11 et le cadre métallique inférieur 12) et la partie supérieure du boîtier (qui comprend la feuille supérieure 15 et le cadre métallique supérieur 16), après la formation des joints hermétiques entre les feuilles et les cadres métalliques, mais avant la formation du deuxième joint hermétique entre les deux cadres métalliques. Dans le cas représenté à la figure 3A, le joint hermétique entre la feuille inférieure 11 et la partie interne 14 du cadre métallique inférieur 12 a été formé par brasage, via un joint de brasure 31 ; de même, le joint hermétique entre la feuille supérieure 15 et la partie interne 18 du cadre métallique supérieur 16 a été formé par brasage, via un joint de brasure 32.

Avantageusement, des éléments - composants, ou dispositifs (et en particulier des composants électroniques) - à encapsuler dans un boîtier hermétique selon l'invention sont positionnés entre la feuille inférieure 11 et la feuille supérieure 15 seulement après la formation du joint hermétique entre les feuilles et les cadres métalliques. En effet, le procédé de formation du joint hermétique entre les feuilles et les cadres métalliques implique généralement une étape dans un four à haute température, ce qui est susceptible de dégrader les éléments à encapsuler.

La figure 3B représente la formation dudit deuxième joint hermétique entre les deux cadres métalliques par soudage au faisceau laser. Le joint hermétique est formé entre la partie externe 13 du cadre métallique inférieur 12 et la partie externe 17 du cadre métallique supérieur 16, par apport localisé de chaleur dans cette zone. Le faisceau laser est représenté schématiquement par deux flèches en trait pointillé, pénétrant dans la partie externe 17 du cadre métallique supérieur 16 et la partie externe 13 du cadre métallique inférieur 12. Etant donné que les parties externes des cadres métalliques 12 et 16 sont positionnées en totalité à l'extérieur du périmètre délimité par les feuilles 11 et 15, le procédé de soudage ne dégrade pas thermiquement ni les joints hermétiques précédemment formés entre les feuilles et les cadres métalliques (notamment les joints de brasure 31 et 32), ni les éléments à encapsuler précédemment positionnés entre les deux feuilles.

Les éléments à encapsuler peuvent faire l'objet d'une « pré-encapsulation », c'est-à-dire d'un enrobage par un matériau polymère préalablement à la formation du deuxième joint hermétique.

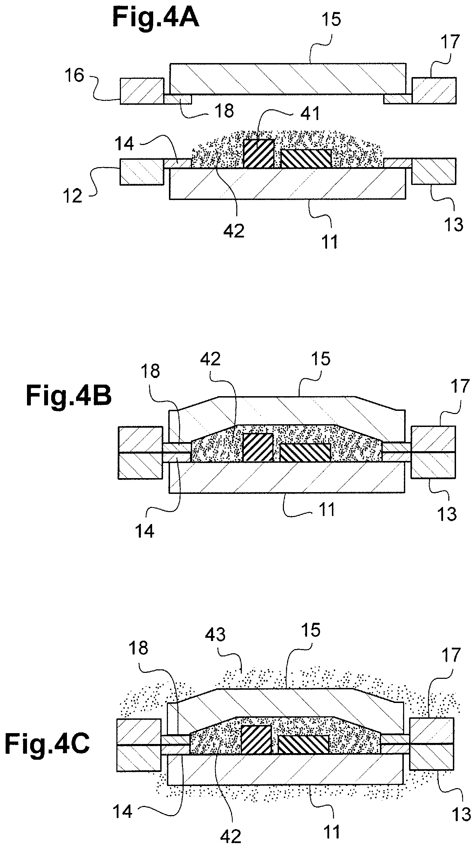

La figure 4A représente la partie inférieure et la partie supérieure du boîtier, après la formation du joint hermétique entre les feuilles et les cadres métalliques, mais avant la formation du joint hermétique entre les deux cadres métalliques. Des éléments 41 à encapsuler sont fixés sur la feuille inférieure 11, éventuellement par l'intermédiaire d'une couche diélectrique si ladite feuille inférieure est métallique. En particulier, la feuille inférieure 11 peut constituer un substrat sur lequel sont assemblés des composants 41 par des techniques de type à puce retournée (« *flip chip* » en anglais) ou soudure de fils (« *wire bonding* » en anglais). Les composants 41 peuvent être des circuits intégrés (de préférence sur substrat de silicium aminci), des composants passifs discrets (notamment des condensateurs ou des bobines), des batteries en couches minces (sur substrat de silicium aminci, ou sur feuille ultrafine en polymère, céramique, métal ou verre), des modules photovoltaïques en couches minces (sur feuille ultrafine en polymère, céramique, métal ou verre), des modules photovoltaïques en silicium cristallin (de préférence sur substrat de silicium aminci), des diodes électroluminescentes (sur substrat aminci), ou tout autre composant électronique, photonique, de récupération d'énergie, ou de stockage de l'énergie.

Les composants 41 sont recouverts d'une couche en polymère 42, dite de pré-encapsulation. Le rôle de la couche de pré-encapsulation 42 est de protéger mécaniquement les composants 41. Avantageusement, l'épaisseur maximale de la couche de pré-encapsulation 42 est inférieure à 300 µm. Avantageusement, et comme représenté à la figure 4A, l'épaisseur de la couche de pré-encapsulation 42 dans les zones situées à proximité de la partie interne 14 du cadre métallique inférieur 12 est inférieure ou environ égale à la somme e_{int-CI} + e_{int-CS}. Avantageusement, la pente du relief de la couche de pré-encapsulation 42 est faible. Plus spécifiquement, la pente du relief de la couche de pré-encapsulation 42 est partout inférieure à 20%. La couche de pré-encapsulation 42 est par exemple à base de silicones ou de polyuréthanes. Ces polymères présentent l'avantage d'être flexibles, facilement mis en forme par des procédés de moulage, et couramment utilisés dans les implants médicaux. La couche de pré-encapsulation 42 peut être formée par un procédé de moulage, de dépôt ou de laminage.

La figure 4B représente le boitier, après la formation du joint hermétique entre les deux cadres métalliques. Avantageusement, la feuille supérieure 15 se déforme légèrement pour épouser de manière conforme les reliefs de la couche de pré-encapsulation 42. Ceci est rendu possible par le fait que la pente du relief de la couche de pré-encapsulation 42 soit faible, et par le fait que la feuille supérieure 15 soit ultrafine. Une telle configuration limite considérablement le risque d'endommagement de la feuille supérieure 15 pendant et après le procédé de formation du joint hermétique entre les deux cadres métalliques.

Avantageusement, la « surface libre » de la feuille supérieure 15 (c'est-à-dire la surface centrale de la feuille supérieure 15 qui ne forme pas de joint hermétique avec la partie interne 18 du cadre métallique supérieur 16) est légèrement supérieure à la «surface libre » de la feuille inférieure 11 (c'est-à-dire la surface centrale de la feuille inférieure 11 qui ne forme pas de joint hermétique avec la partie interne 14 du cadre métallique inférieur 12). Ceci permet à la feuille supérieure 15 de se déformer légèrement pour épouser de manière conforme les reliefs de la couche de pré-encapsulation 42.

Le dispositif médical implantable encapsulé représenté schématiquement à la figure 4B se présente sous la forme d'un objet ultrafin et mécaniquement conformable. L'épaisseur de l'objet :
- au niveau de l'empilement 13/17 est de préférence inférieure à 100 µm (si l'épaisseur de chacune des parties externes des cadres métalliques est inférieure à 50 µm), et peut être de l'ordre de 60 µm (si l'épaisseur de chacune des parties externes des cadres métalliques est de l'ordre de 30 µm),
- au niveau de l'empilement 11/14/18/15 est de préférence inférieure à 150 µm (si l'épaisseur de chacune des feuilles est inférieure à 50 µm et si l'épaisseur de chacune des parties internes des cadres métalliques est inférieure à 25 µm), et peut être de l'ordre de 100 µm (si l'épaisseur de chacune des feuilles est de l'ordre de 40 µm et si l'épaisseur de chacune des parties internes des cadres métalliques est de l'ordre de 10 µm),
- au niveau de l'empilement 11/42/15 est de préférence inférieure à 400 µm (si l'épaisseur de chacune des feuilles est inférieure à 50 µm et si l'épaisseur maximale de la couche de pré-encapsulation est inférieure à 300 µm), et peut être de l'ordre de 250 µm (si l'épaisseur de chacune des feuilles est de l'ordre de 40 µm et si l'épaisseur maximale de la couche de pré-encapsulation est de l'ordre de 170 µm).

Ainsi l'épaisseur de l'objet au niveau de sa zone la plus épaisse (c'est-à-dire au niveau de l'empilement 11/42/15) peut être de l'ordre de 250 µm. En termes de flexibilité mécanique, la flexibilité de l'objet est limitée par les feuilles (qui sont des pièces en céramique, métal ou verre) et les cadres métalliques. La flexibilité n'est pas limitée par la couche de pré-encapsulation 42, car il s'agit d'une couche en polymère qui demeure flexible même pour de fortes épaisseurs. La flexibilité sera donc essentiellement limitée par l'épaisseur de l'empilement 11/14/18/15, qui peut être de l'ordre de 100 µm comme détaillé ci-dessus. Une telle épaisseur est suffisamment faible pour assurer une bonne flexibilité mécanique à l'objet final.

Le boîtier peut aussi être enrobé par un matériau polymère, après la formation du deuxième joint hermétique (« post-encapsulation »).

La figure 4C représente une couche en polymère 43, dite de post-encapsulation, qui enrobe la feuille inférieure 11, la feuille supérieure 15, et les joints hermétiques formés latéralement entre ces deux feuilles. Un premier rôle de la couche de post-encapsulation 43 est de protéger mécaniquement l'ensemble du dispositif et du boîtier, notamment la feuille inférieure 11 et la feuille supérieure 15. Un deuxième rôle de la couche de post-encapsulation 43 est d'obtenir un dispositif final dont les bords ne soient pas tranchants. Avantageusement, l'épaisseur maximale de la couche de post-encapsulation 43 est inférieure à 300 µm. La couche de post-encapsulation 43 est par exemple à base de silicones ou de polyuréthanes. La couche de post-encapsulation 43 peut être formée par un procédé de moulage, de dépôt ou de laminage.

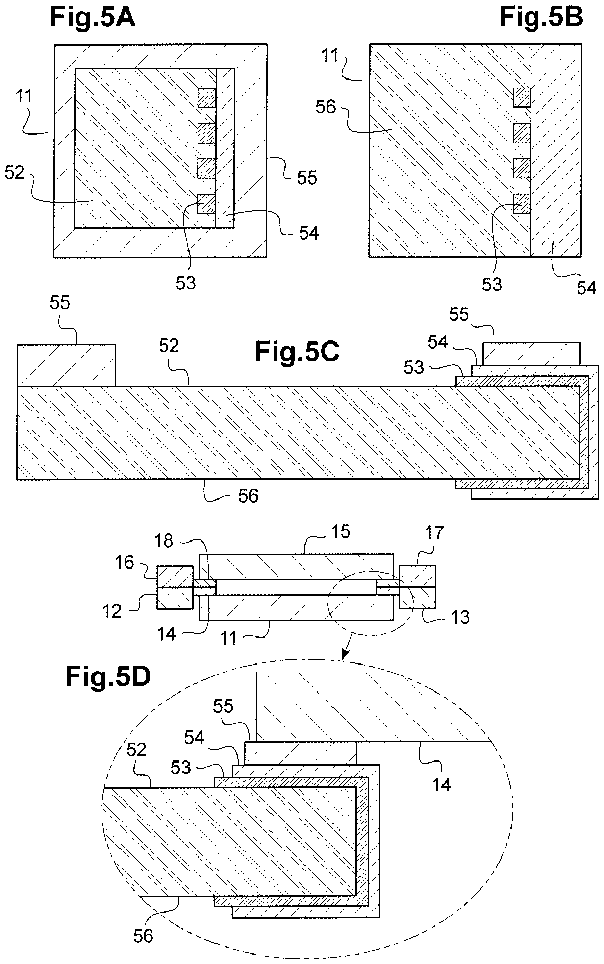

Des dispositifs ou composants électroniques encapsulés à l'intérieur d'un boîtier selon l'invention doivent le plus souvent pouvoir communiquer avec l'extérieur au moyen d'électrodes. Par exemple, un stimulateur neuronal ou cardiaque doit pouvoir délivrer des impulsions à des tissus, directement ou par l'intermédiaire de sondes. Il est donc nécessaire de permettre la sortie d'une ou plusieurs pistes électriques formant électrodes ou plages de connexion, et cela sans compromettre l'herméticité du boîtier. Une possible solution à ce problème est illustrée par la figure 5.

La figure 5A (vue de dessus), la figure 5B (vue de dessous) et la figure 5C (vue en coupe selon l'axe représenté en pointillé sur la figure 5A) représentent la sortie d'électrodes 53 au niveau de la feuille inférieure 11. Comme mentionné précédemment, la feuille inférieure 11 est avantageusement une pièce en céramique, en métal ou en verre. Dans le cas où la feuille inférieure 11 est en métal, elle doit être recouverte sur sa face inférieure et sa face supérieure ainsi que sur ses tranches par une couche mince électriquement isolante, par exemple une couche mince d'alumine déposée par dépôt chimique en phase vapeur. Des pistes électriquement conductrices 53 sont formées sur l'un des bords de la feuille inférieure 11, de manière à ce que les pistes conductrices 53 relient la face supérieure 52 et la face inférieure 56 de la feuille inférieure 11, via la tranche de la feuille inférieure 11. La largeur des pistes conductrices 53 est avantageusement comprise entre 10 µm et 1 mm. L'épaisseur des pistes conductrices 53 est avantageusement comprise entre 10 nm et 1 µm. Les pistes conductrices 53 sont par exemple des couches minces métalliques (or, platine, etc.) déposées par dépôt physique en phase vapeur, dépôt chimique en phase vapeur, électrodépôt, enduction ou impression. L'étape de dépôt peut être associée à une étape de structuration, par exemple par photolithographie, gravure chimique, gravure plasma, gravure mécanique ou gravure laser. Les pistes conductrices 53 sont recouvertes de manière conforme par une couche mince électriquement isolante 54. Seules les extrémités des pistes conductrices 53 (au niveau de la face supérieure 52 et de la face inférieure 56 de la feuille inférieure 11) ne sont pas recouvertes par la couche mince isolante 54. L'épaisseur de la couche mince isolante 54 est avantageusement comprise entre 10 nm et 1 µm. La couche mince isolante 54 est par exemple une couche mince de silice, nitrure de silicium, alumine ou zircone déposée par dépôt physique en phase vapeur, par dépôt chimique en phase vapeur, enduction ou impression. L'étape de dépôt peut être associée à une étape de structuration. Un cadre électriquement isolant 55 est formé sur les bords de la face supérieure 52 de la feuille inférieure 11 ; il a une fonction de planarisation desdits bords, afin que la partie interne 14 du cadre métallique inférieur puisse être positionné sur une surface plane. Le cadre isolant 55 recouvre partiellement la couche mince isolante 54 et les pistes conductrices 53. L'épaisseur du cadre isolant 55 est avantageusement comprise entre 100 nm et 50 µm. Le cadre isolant 55 peut être par exemple une couche de silice, de nitrure de silicium, d'alumine ou de zircone déposée par dépôt physique en phase vapeur, dépôt chimique en phase vapeur, enduction ou impression. L'étape de dépôt peut être associée à une étape de structuration. Un matériau isolant jouant le rôle de barrière de diffusion, telle qu'une céramique dense , est particulièrement avantageux, afin d'éviter la diffusion d'espèces métalliques à travers le cadre isolant 55 lors de la formation du joint hermétique entre la feuille inférieure 11 et la partie interne 14 du cadre métallique inférieur 12. Alternativement, le cadre isolant 55 peut être une pièce en céramique (de préférence en zircone ZrO₂ stabilisée à l'oxyde d'yttrium Y₂O₃ à 3% molaire ou plus, ou en zircone ZrO₂ stabilisée à l'oxyde de cérium CeO₂), assemblée par cofrittage avec la feuille inférieure 11.

La figure 5D (vue en coupe) représente schématiquement le boîtier une fois les différentes pièces assemblées (feuille inférieure 11 avec la sortie d'électrodes 53, cadre métallique inférieur 12, feuille supérieure 15, cadre métallique supérieur 16).

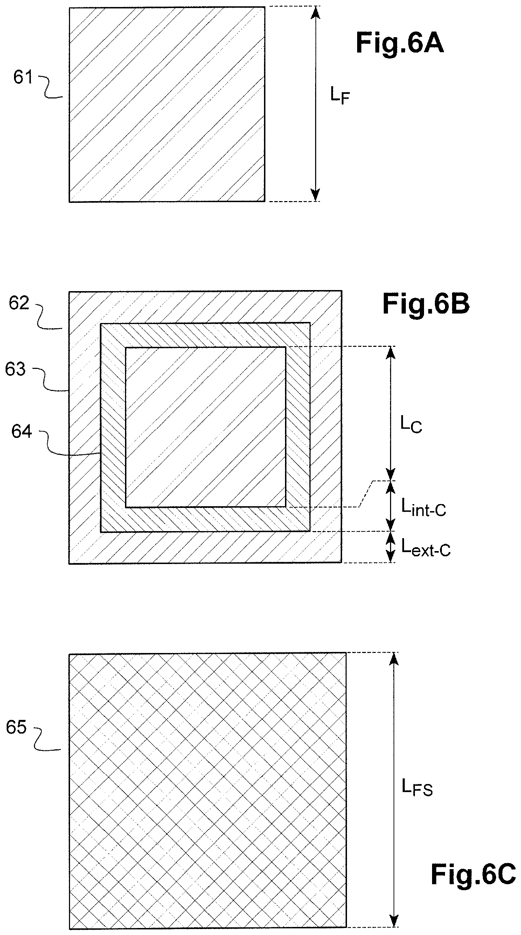

Les figures 6 à 8 illustrent un boîtier selon un deuxième mode de réalisation de l'invention et les différentes étapes de son procédé de fabrication. Ce deuxième mode de réalisation se différencie du premier mode de réalisation décrit ci-dessus en ce que le deuxième élément servant de couvercle est constitué par la seule deuxième feuille (ou feuille supérieure), qui est réalisée en métal, le deuxième cadre (ou cadre supérieur) étant absent.

La figure 6 (vue de dessus) représente schématiquement les différentes pièces d'un tel boîtier, avant leur assemblage.

La figure 6A représente la feuille inférieure 61. Dans le cas représenté à la figure 6A, la feuille inférieure 61 est une pièce de forme carrée, de côté noté L_{F}, avantageusement compris entre 5 mm et 10 cm. D'autres formes sont envisageables, en particulier une forme circulaire. L'épaisseur de la feuille inférieure 61, notée e_{FI}, est avantageusement inférieure à 500 µm, de préférence inférieure à 50 µm. La feuille inférieure 61 est avantageusement une pièce en céramique (de préférence en zircone ZrO₂ stabilisée à l'oxyde d'yttrium Y₂O₃ à 3% molaire ou plus, ou en zircone ZrO₂ stabilisée à l'oxyde de cérium CeO₂), ou en métal (de préférence en titane ou en alliage de titane). La feuille inférieure 61 peut être également une pièce en verre.

La figure 6B représente le seul cadre métallique du boîtier qui, dans un souci de cohérence terminologique avec le premier mode de réalisation sera appelé « premier cadre métallique » ou « cadre métallique inférieur» 62. Dans le mode de réalisation exemplaire considéré ici, le cadre métallique inférieur 62 est une pièce de forme carrée, et présentant une ouverture centrale de forme carrée de côté noté L_{C}, avantageusement compris entre 5 mm et 10 cm. Comme dans le premier mode de réalisation de l'invention, le cadre métallique inférieur 62 comprend deux parties :
- une partie externe 63, de largeur notée L_{ext-C} (avantageusement comprise entre 500 µm et 1 cm), et d'épaisseur notée e_{ext-CI} (avantageusement inférieure à 500 µm, de préférence inférieure à 50 µm) ;
- une partie interne 64, de largeur notée L_{int-C} (avantageusement comprise entre 500 µm et 1 cm), et d'épaisseur notée e_{int-CI} inférieure ou égale à e_{ext-CI}.

Dans le mode de réalisation exemplaire considéré ici, la partie externe 63 et la partie interne 64 constituent une seule et unique pièce monolithique 62. Le cadre métallique inférieur 62 est avantageusement une pièce en titane ou en alliage de titane.

La figure 6C représente la feuille métallique supérieure 65. Dans le cas représenté à la figure 6C, la feuille métallique supérieure 65 est une pièce de forme carrée, de côté noté L_{FS}, avantageusement compris entre 5 mm et 10 cm. Le côté L_{FS} de la feuille métallique supérieure 65 est plus grand que le côté L_{F} de la feuille inférieure 61, afin de permettre le positionnement de la partie externe 63 du cadre métallique inférieur 62 à la verticale de la périphérie de la feuille métallique supérieure 65, puis de former un joint hermétique entre la partie externe 63 du cadre métallique inférieur 62 et la périphérie de la feuille métallique supérieure 65 (voir ci-dessous). L'épaisseur de la feuille métallique supérieure 65, notée e_{FS}, est avantageusement inférieure à 500 µm, de préférence inférieure à 50 µm. La feuille métallique supérieure 65 est avantageusement une pièce en titane ou en alliage de titane.

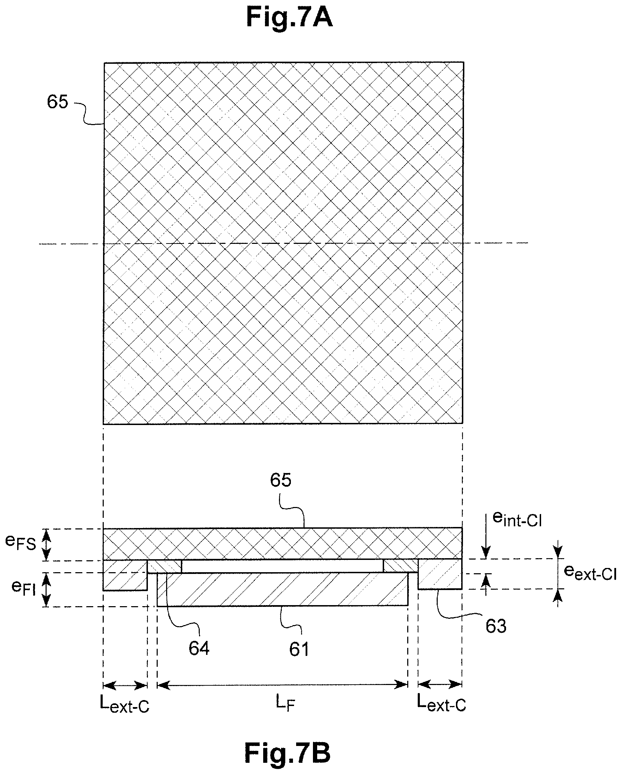

La figure 7 représente schématiquement le boîtier de la figure 6 une fois les différentes pièces assemblées (feuille inférieure 61, cadre métallique inférieur 62, feuille métallique supérieure 65). La figure 7A est une vue de dessus, alors que la figure 7B est une vue en coupe selon l'axe représenté en pointillé sur la figure 7A. La partie externe 63 du cadre métallique inférieur 62 est positionnée en totalité à l'extérieur du périmètre délimité par la feuille inférieure 61, et la partie interne 64 du cadre métallique inférieur 62 est positionnée en partie à l'intérieur du périmètre délimité par la feuille inférieure 61. Un premier joint hermétique est formé entre la feuille inférieure 61 et la partie interne 64 du cadre métallique inférieur 62. Avantageusement, ce joint hermétique est formé par brasage. Alternativement, ce joint hermétique peut être formé par soudage par diffusion à l'état solide ou par cofrittage. Un deuxième joint hermétique est formé entre la partie externe 63 du cadre métallique inférieur 62 et la périphérie de la feuille métallique supérieure 65. Avantageusement, ce joint hermétique est formé par soudage au faisceau laser. D'autres procédés de soudage avec apport localisé de chaleur peuvent être utilisés, comme le soudage par faisceau d'électrons, ou le soudage par résistance (effet Joule).

Comme cela a été expliqué au sujet du premier mode de réalisation, il est avantageux que la partie externe 63 du cadre métallique inférieur 62 soit positionnée en totalité à l'extérieur du périmètre délimité par la feuille inférieure 61 ; et que l'épaisseur e_{ext-CI} de la partie externe 63 du cadre métallique inférieur 62 soit supérieure ou égale à l'épaisseur e_{int-CI} de la partie interne 64.

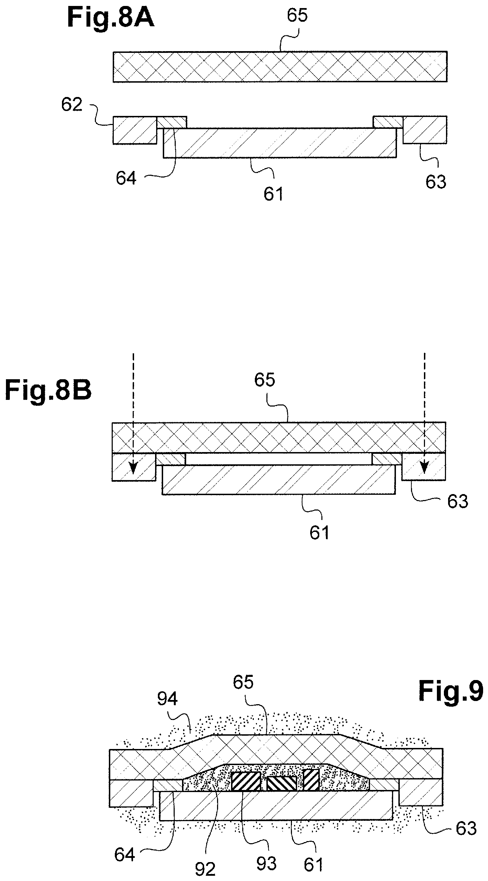

La figure 8 (vue en coupe) représente schématiquement le procédé de soudage au faisceau laser pour la formation du joint hermétique entre la partie externe 63 du cadre métallique inférieur 62 et la périphérie de feuille métallique supérieure 65.

La figure 8A représente la partie inférieure du boîtier (qui comprend la feuille inférieure 61 et le cadre métallique inférieur 62) et la partie supérieure du boîtier (qui comprend la feuille métallique supérieure 65), après la formation du joint hermétique entre la feuille inférieure 61 et le cadre métallique inférieur 62, mais avant la formation du joint hermétique entre le cadre métallique inférieur 62 et la feuille métallique supérieure 65. Comme cela a été expliqué au sujet du premier mode de réalisation, il est avantageux que des éléments - composants, ou dispositifs (et en particulier des composants électroniques) - à encapsuler dans un boîtier hermétique selon l'invention soient positionnés entre la feuille inférieure 61 et la feuille supérieure 65 seulement après la formation du joint hermétique entre la feuille inférieure 61 et le cadre métallique inférieur 62. En effet, la formation de ce joint implique généralement une étape dans un four à haute température, ce qui est susceptible de dégrader les éléments à encapsuler.

La figure 8B représente la formation du deuxième joint hermétique entre le cadre métallique inférieur 62 et la feuille métallique supérieure 65 par soudage au faisceau laser. Le joint hermétique est formé entre la partie externe 63 du cadre métallique inférieur 62 et la périphérie de la feuille métallique supérieure 65, par apport localisé de chaleur dans cette zone. Le faisceau laser est représenté schématiquement par deux flèches en trait pointillé, pénétrant dans la périphérie de la feuille métallique supérieure 65 et la partie externe 63 du cadre métallique inférieur 62. Comme dans le cas du premier mode de réalisation, étant donné que la partie externe 63 du cadre métallique inférieur 62 est positionnée en totalité à l'extérieur du périmètre délimité par la feuille inférieure 61, le procédé de soudage ne dégrade pas thermiquement ni le joint hermétique précédemment formé entre la feuille inférieure 61 et le cadre métallique inférieur 62, ni les éléments à encapsuler précédemment positionnés entre les deux feuilles.

De la même manière que dans le premier mode de réalisation, l'encapsulation peut être complétée par une pré-encapsulation 92 protégeant mécaniquement les éléments 93 et/ou une post-encapsulation 94, comme représenté à la figure 9 (vue en coupe).

De la même manière que dans le premier mode de réalisation, une sortie hermétique d'une ou plusieurs électrodes peut être aménagée.

L'invention admet diverses variantes et améliorations.

Par exemple, un dessicant peut être introduit dans le volume interne défini par le boîtier, notamment par dépôt d'une couche mince d'un gel de silice sur la face interne de l'une des feuilles, ou par utilisation d'un polymère de pré-encapsulation chargé avec des particules (en particulier des nanoparticules) de silice.

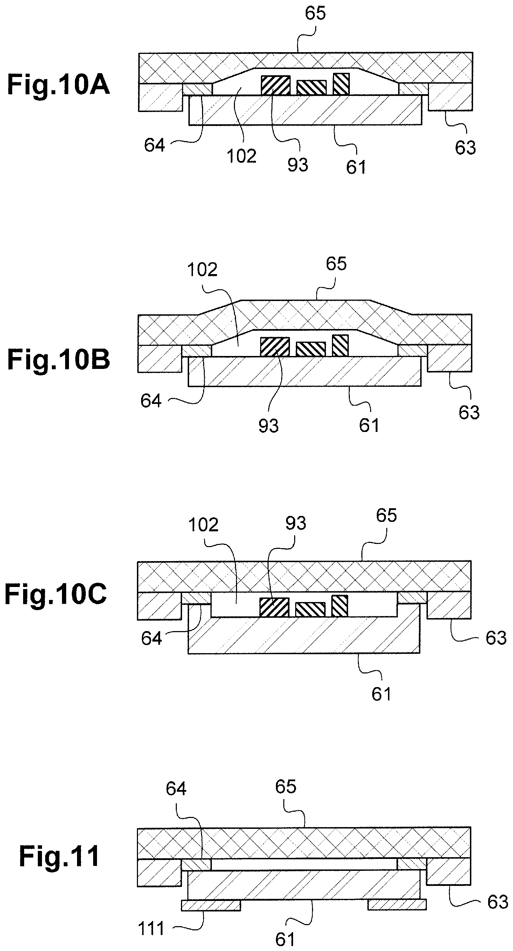

Les feuilles peuvent présenter un relief contrôlé, notamment afin de définir une cavité. Par exemple, comme représenté schématiquement à la figure 10A dans le cas du deuxième mode de réalisation, la feuille métallique supérieure 65 peut présenter une forme concave. Ceci permet, après assemblage des différentes pièces du boîtier, de définir une cavité 102 contenant les composants 93.

Dans un autre exemple, représenté schématiquement à la figure 10B dans le cas du deuxième mode de réalisation, la feuille métallique supérieure 65, initialement plane, a été mise en forme par emboutissage ou hydroformage ou tout autre procédé mécanique. Comme on peut le constater, les faces interne et externe de la feuille 65 sont toutes deux de forme concave. Ceci permet, après assemblage des différentes pièces du boitier, de définir une cavité 102 contenant les composants 93. L'épaisseur de l'objet décrit à la figure 10B dans sa zone la plus épaisse (c'est-à-dire au niveau de l'empilement 61/102/65) peut être par exemple de l'ordre de 3 mm (si l'épaisseur de chacune des feuilles 61 et 65 est de l'ordre de 500 µm, et si la hauteur maximale de la cavité 102 est de l'ordre de 2 mm). A l'autre extrême de la gamme d'épaisseur avantageusement considérée dans la présente invention, l'épaisseur de l'objet décrit à la figure 10B dans sa zone la plus épaisse (c'est-à-dire au niveau de l'empilement 61/102/65) peut être de l'ordre de 250 µm (si l'épaisseur de chacune des feuilles 61 et 65 est de l'ordre de 40 µm, et si la hauteur maximale de la cavité 102 est de l'ordre de 170 µm).

Dans un autre exemple, représenté schématiquement à la figure 10C dans le cas du deuxième mode de réalisation, la feuille inférieure 61 peut présenter une forme en U. Dans le cas où la feuille inférieure 61 est une pièce en céramique, une telle forme en U peut être obtenue par co-frittage d'une feuille plane en céramique et d'un cadre en céramique. La forme en U de la feuille inférieure 61 permet, après assemblage des différentes pièces du boitier, de définir une cavité 102 contenant les composants 93. Dans le cas où la feuille inférieure 61 est une pièce en céramique, il est préférable de définir la cavité 102 en donnant une forme en U à la feuille inférieure 61, plutôt que de simplement augmenter l'épaisseur de la partie interne 64 du cadre métallique inférieur 62 ; en effet, augmenter l'épaisseur de la partie interne 64 du cadre métallique inférieur 62 a pour conséquence d'augmenter la quantité de métal dans le boitier, ce qui peut poser des problèmes dans le cas d'applications implantables en matière de compatibilité du boitier avec l'imagerie par résonance magnétique (IRM).

Finalement, la feuille métallique supérieure 65 peut présenter une forme concave, uniquement sur sa face interne ou à la fois sur ses faces interne et externe et/ou la feuille inférieure 61 peut présenter une forme en U.

Les variantes des figures 10A, 10B et 10C concernent également le premier mode de réalisation.

Dans le cas d'une feuille en céramique ou en verre, un cadre métallique de protection peut être ajouté sur la face externe, afin de renforcer la résistance mécanique. Par exemple, comme représenté à la figure 11 dans le cas du deuxième mode de réalisation, un cadre métallique de protection 111 est fixé sur la face inférieure de la feuille inférieure 61. L'ouverture centrale du cadre métallique de protection 111 peut être plus ou moins grande selon l'application visée. Cette variante concerne également le premier mode de réalisation.

Dans le cas d'une feuille en céramique, un dépôt de couches minces peut être effectué sur la face externe, afin de renforcer la résistance contre les attaques par les tissus ou fluides biologiques, et de limiter le vieillissement de la céramique. En effet, les céramiques telles que la zircone stabilisée à l'oxyde d'yttrium sont susceptibles de vieillir dans des environnements humides. Les couches minces peuvent être par exemple à base de métaux (titane, or, platine, etc.), d'oxydes (alumine, silice, etc.) ou de polymères hydrophobes (polytétrafluoroéthylène, etc.).

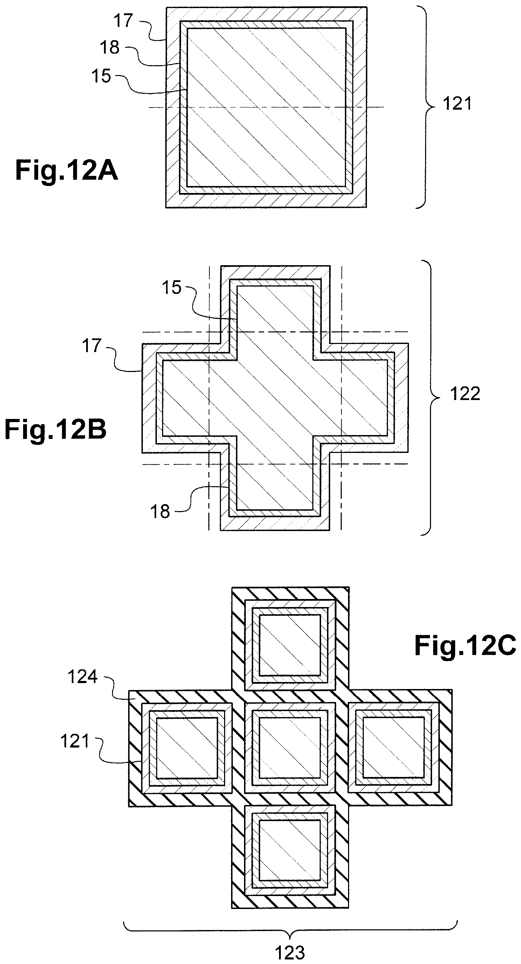

La figure 12A (vue de dessus) représente un boitier 121 avec une forme géométrique simple. Avec une telle forme géométrique, le boitier peut être courbé selon un axe (par exemple, l'axe représenté en pointillés sur la figure 12A), ce qui permet de positionner de manière conforme le boitier sur des objets de type cylindrique. Cependant, il est plus difficile de courber le boitier 121 selon plusieurs axes, car ceci engendre des contraintes mécaniques fortes susceptibles d'endommager le boitier. Ainsi, il est plus difficile de positionner de manière conforme le boitier 121 sur des objets non cylindriques (par exemple des objets de type sphérique).

Si un boitier présente une forme géométrique plus complexe, alors il peut être courbé selon un ou plusieurs axes (en particulier plusieurs axes non parallèles entre eux), sans engendrer de contraintes mécaniques fortes. Le boitier peut par exemple présenter une forme de croix, donc plusieurs branches, avec au moins un axe de courbure potentiel au niveau de chaque branche. Ceci autorise alors de positionner de manière conforme le boitier sur des objets de formes variées qui ne sont pas nécessairement cylindriques (par exemple des objets de type sphérique). A titre d'exemple, la figure 12B (vue de dessus) représente un boitier 122 possédant une forme de croix avec quatre branches. Avec une telle forme géométrique, le boitier 122 peut être courbé selon au moins quatre axes (par exemple, les axes représentés en pointillés sur la figure 12B).

Il est possible de fabriquer un assemblage ou dispositif de plusieurs boitiers, cet assemblage possédant un degré de conformabilité ou flexibilité mécanique supérieur à celui d'un boitier unique (pour une forme et des dimensions données). A titre d'exemple, la figure 12C représente un assemblage 123 de plusieurs boitiers 121. L'assemblage 123 possède une forme et des dimensions similaires à celles du boitier 122 représenté à la figure 12B. Cependant, l'assemblage 123 possède potentiellement un degré de conformabilité ou flexibilité mécanique supérieur à celui du boitier 122. En effet, les boitiers 121 inclus dans l'assemblage 123 sont reliés mécaniquement par un support commun flexible 124 par exemple réalisé en polymère. Ce polymère peut être biocompatible, par exemple un polymère à base de silicone ou de polyuréthane. Le support commun flexible 124 peut être par exemple une feuille en polymère sur laquelle sont collés (sur une ou deux faces) les boitiers 121 ; le support 124 peut être également une pièce en polymère surmoulée enrobant partiellement ou complètement les boitiers 121. En plus de son rôle mécanique, le support 124 peut jouer un rôle d'interconnexion électrique. Ainsi, le support 124 peut inclure des pistes ou fils métalliques permettant d'interconnecter électriquement les différents boîtiers 121 ; le support 124 peut aussi inclure des connecteurs électriques (permettant de connecter l'assemblage 123 à d'autres composants). Enfin, le support 124 peut inclure des composants électroniques qu'il n'est pas nécessaire d'encapsuler dans les boitiers 121 (par exemple, une bobine ou une antenne, permettant de recevoir ou émettre des informations ou de l'énergie).

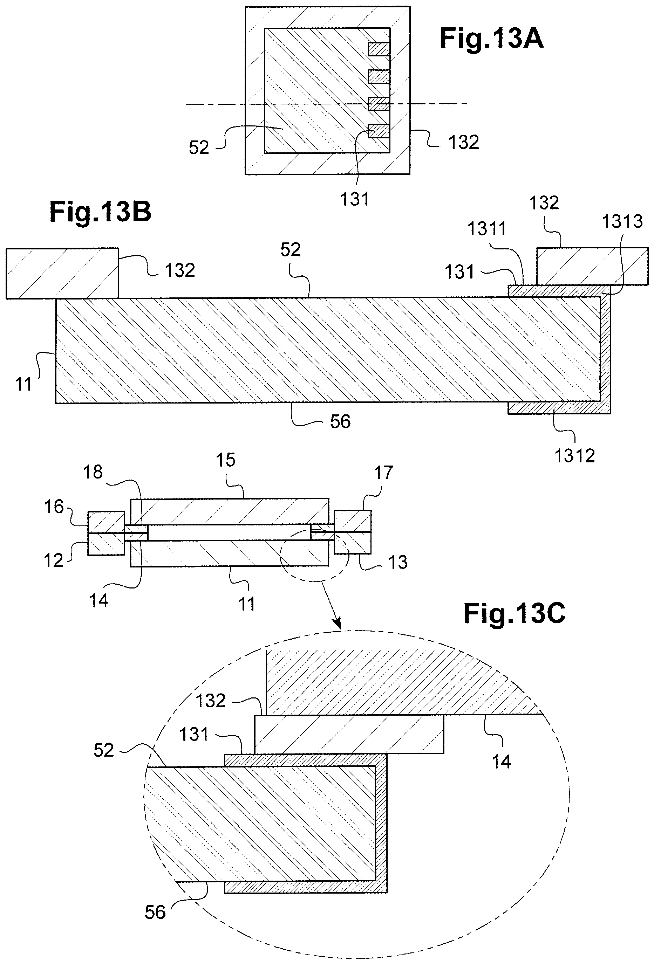

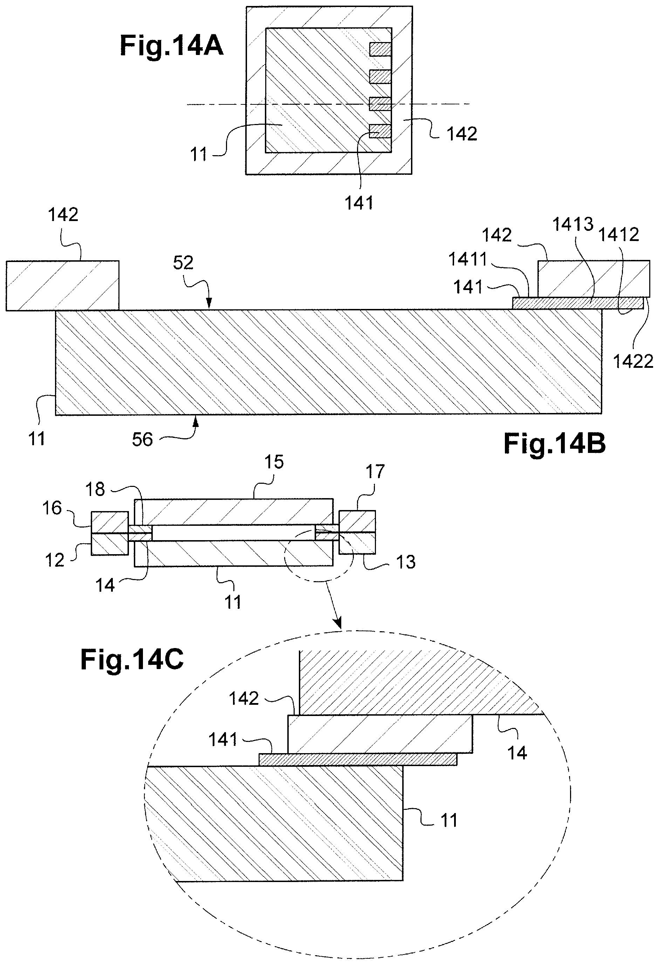

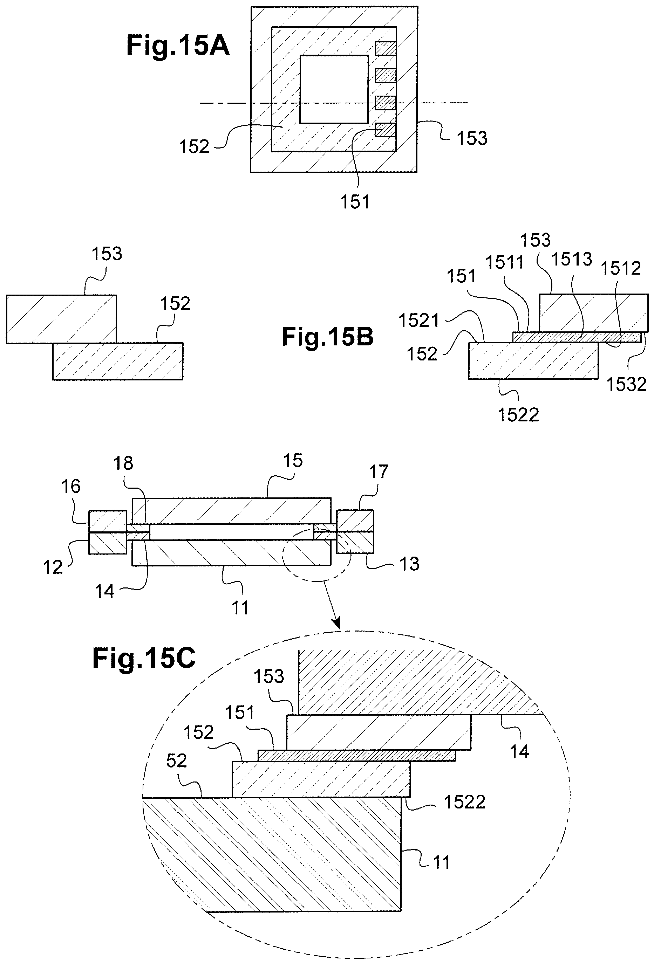

Les figures 13, 14 et 15 présentent des montages alternatifs pour permettre la sortie d'une ou plusieurs pistes électriques formant électrodes ou plages de connexion, et cela sans compromettre l'herméticité du boitier. Les figures 13 et 14 décrivent le cas où la feuille inférieure 11 est une pièce en céramique, et la figure 15 décrit le cas où la feuille inférieure 11 est une pièce en métal.

La figure 13A (vue de dessus) et la figure 13B (vue en coupe selon l'axe représenté en pointillé sur la figure 13A) représentent la sortie d'électrodes 131 au niveau d'un assemblage incluant la feuille inférieure 11 et un cadre additionnel 132. Dans le cas considéré ici, la feuille inférieure 11 est une pièce en céramique, de préférence en zircone ZrO₂ stabilisée à l'oxyde d'yttrium Y₂O₃ à 3% molaire ou plus, ou en zircone ZrO₂ stabilisée à l'oxyde de cérium CeO₂. Le cadre additionnel 132 est également une pièce en céramique. Les électrodes 131 sont des pistes métalliques, de préférence en platine ou en alliage de platine. Les pistes métalliques 131 forment des traversées hermétiques céramique-métal entre la feuille inférieure 11 et le cadre 132. Chaque piste métallique 131 relie la face supérieure 52 et la face inférieure 56 de la feuille inférieure 11, via la tranche de la feuille inférieure 11.

Chaque piste métallique 131 présente :
- une portion 1313 prise en sandwich entre la feuille inférieure 11 et le cadre 132,
- une première extrémité 1311 accessible sur la face supérieure 52 de la feuille inférieure 11,
- une deuxième extrémité 1312 accessible sur la face inférieure 56 de la feuille inférieure 11.

L'assemblage incluant les pistes métalliques 131, la feuille inférieure 11 et le cadre additionnel en céramique 132 est de préférence élaboré par co-frittage.

La figure 13C (vue en coupe) représente schématiquement le boitier une fois les différentes pièces assemblées. Un premier joint hermétique est formé entre le cadre additionnel 132 et la partie interne 14 du cadre métallique inférieur 12. Avantageusement, ce premier joint est formé par brasage.

La figure 14A (vue de dessus) et la figure 14B (vue en coupe selon l'axe représenté en pointillé sur la figure 14A) représentent la sortie d'électrodes 141 au niveau d'un assemblage incluant la feuille inférieure 11 et un cadre additionnel 142. Dans le cas considéré ici, la feuille inférieure 11 est une pièce en céramique, de préférence en zircone ZrO₂ stabilisée à l'oxyde d'yttrium Y₂O₃ à 3% molaire ou plus, ou en zircone ZrO₂ stabilisée à l'oxyde de cérium CeO₂. Le cadre additionnel 142 est également une pièce en céramique. Les électrodes 141 sont des pistes métalliques, de préférence en platine ou en alliage de platine. Les pistes métalliques 141 forment des traversées hermétiques céramique-métal entre la feuille inférieure 11 et le cadre 142.

Chaque piste métallique 141 présente :
- une portion 1413 prise en sandwich entre la feuille inférieure 11 et le cadre additionnel 142 ;
- une première extrémité 1411 accessible sur la face supérieure 52 de la feuille inférieure 11 ;
- une deuxième extrémité 1412 accessible sur la face inférieure 1422 du cadre additionnel 142.

L'assemblage incluant les pistes métalliques 141, la feuille inférieure 11 et le cadre 142 est de préférence élaboré par co-frittage.

La figure 14C (vue en coupe) représente schématiquement le boitier une fois les différentes pièces assemblées. Un premier joint hermétique est formé entre le cadre 142 et la partie interne 14 du cadre métallique inférieur 12. Avantageusement, ce premier joint est formé par brasage.

La figure 15A (vue de dessus) et la figure 15B (vue en coupe selon l'axe représenté en pointillé sur la figure 15A) représentent la sortie d'électrodes 151 au niveau d'un assemblage incluant un premier cadre additionnel 152 et un deuxième cadre additionnel 153. Les deux cadres 152, 153 sont des pièces en céramique, de préférence en zircone ZrO₂ stabilisée à l'oxyde d'yttrium Y₂O₃ à 3% molaire ou plus, ou en zircone ZrO₂ stabilisée à l'oxyde de cérium CeO₂. Les électrodes 151 sont des pistes métalliques, de préférence en platine ou en alliage de platine. Les pistes métalliques 151 forment des traversées hermétiques céramique-métal entre le cadre 152 et le cadre 153.

Chaque piste métallique 151 présente :
- une portion 1513 prise en sandwich entre le premier cadre additionnel 152 et le deuxième cadre additionnel 153,
- une première extrémité 1511 accessible sur la face supérieure 1521 du premier cadre additionnel 152,
- une deuxième extrémité 1512 accessible sur la face inférieure 1532 du deuxième cadre additionnel 153.

L'assemblage incluant les pistes métalliques 151, le cadre 152 et le cadre 153 est de préférence élaboré par co-frittage.

La figure 15C (vue en coupe) représente schématiquement le boitier une fois les différentes pièces assemblées. Un premier joint hermétique est formé entre le deuxième cadre additionnel 153 et la partie interne 14 du cadre métallique inférieur 12. Avantageusement, ce premier joint est formé par brasage. Un autre joint hermétique est également formé entre le premier cadre additionnel 152 et la feuille inférieure 11. Dans le cas considéré ici, la feuille inférieure 11 est une pièce en métal. Avantageusement, le joint entre le premier cadre additionnel 152 et la feuille inférieure 11 est formé par brasage, simultanément à la formation du joint entre le deuxième cadre additionnel 153 et la partie interne 14 du cadre métallique inférieur 12.

Une autre méthode pour permettre la sortie d'une ou plusieurs pistes électriques formant électrodes ou plages de connexion consiste à utiliser des traversées hermétiques céramique-métal aménagées au travers de la feuille inférieure 11, dans le cas où cette feuille inférieure est une pièce en céramique. Ces traversées peuvent être élaborées par co-frittage, comme décrit par exemple à la figure 2 dans l'article précité de A. Vanhoestenberghe et al.

Un dispositif de test de l'herméticité du boitier peut être intégré dans le boitier, afin de qualifier de manière non destructive le niveau d'herméticité du boitier fabriqué. Les tests hélium classiquement employés pour tester l'herméticité des boitiers en titane conventionnels peuvent difficilement être utilisés dans la présente invention du fait des faibles volumes du boitier. Afin de tester l'herméticité de boitiers de faibles volumes (inférieurs à 50 mm³), il a été proposé dans le document EP 1 533 270 d'utiliser un élément témoin dont les propriétés optiques ou électriques changent en présence d'un fluide réactif. Ce document propose notamment d'utiliser une couche de cuivre (Cu) pour assurer un contrôle optique. En effet, en présence d'oxygène la couche de Cu s'oxyde, modifiant ainsi ses propriétés optiques. En s'oxydant, la couche de Cu devient transparente (particulièrement dans le proche infrarouge) et il est ainsi possible de quantifier la quantité d'oxygène ayant pénétrée au sein du boitier et de remonter à un taux de fuite du boitier. Ceci nécessite l'utilisation d'une source lumineuse d'intensité connue et d'un capteur afin de quantifier soit le rayonnement transmis au travers de l'ensemble du boitier (ce qui demande que le boitier soit totalement transparent) soit le rayonnement réfléchi. Il est donc avantageux, dans le cas de la présente invention, d'utiliser un capteur présent au sein du boitier pour caractériser l'évolution des propriétés optiques de l'élément témoin déposé par exemple sur la face intérieure d'une feuille en céramique ou en verre. Il sera ainsi possible de transcrire une variation optique de la couche en signal électrique par l'intermédiaire du capteur présent au sein du boitier. Ce capteur pourra être par exemple un photodétecteur ou une cellule photovoltaïques.

## Revendications

1. Boîtier hermétique ultrafin présentant une épaisseur inférieure ou égale à 3 mm, le boîtier hermétique ultrafin comprenant :
- un premier élément en forme de feuille (11, 61), réalisé en au moins un matériau choisi parmi un métal, une céramique et un verre ;
- un deuxième élément (15, 16 ; 65) de dimensions et forme adaptés pour recouvrir ledit premier élément, également réalisé en au moins un matériau choisi parmi un métal, une céramique et un verre ;
- un premier cadre métallique (12, 62) interposé entre ledit premier élément et ledit deuxième élément, comprenant une partie interne (14, 64) positionnée en partie ou en totalité à l'intérieur du périmètre dudit premier élément et une partie externe (13, 63) positionnée en totalité à l'extérieur dudit périmètre dudit premier élément ;
- un premier joint hermétique (31) entre ledit premier élément et ladite partie interne dudit premier cadre métallique ; et
- un deuxième joint hermétique entre ledit deuxième élément et ladite partie externe dudit premier cadre métallique.

2. Boîtier hermétique selon la revendication 1, dans lequel ledit deuxième élément comprend :
- une feuille dite supérieure (15), réalisée en au moins un matériau choisi parmi un métal, une céramique et un verre ;
- un deuxième cadre métallique (16) interposé entre ladite feuille supérieure et ledit premier cadre métallique, comprenant une partie interne (18) positionnée en partie ou en totalité à l'intérieur du périmètre de ladite feuille supérieure et une partie externe (17) positionnée en totalité à l'extérieur dudit périmètre de ladite feuille supérieure ; et
- un troisième joint hermétique (32) entre ladite feuille supérieure et ladite partie interne dudit deuxième cadre métallique ;
ledit deuxième joint hermétique étant entre les parties externes desdits premier et deuxième cadre métallique.

3. Boîtier hermétique selon la revendication précédente, dans lequel ledit troisième joint hermétique est réalisé par une technique choisie parmi : le brasage, le soudage par diffusion à l'état solide et, lorsque ladite feuille supérieure est au moins en partie en céramique, le cofrittage.

4. Boîtier hermétique selon la revendication 1 dans lequel ledit deuxième élément comprend une feuille dite supérieure (65) en métal, directement fixée à la partie externe dudit premier cadre métallique par ledit deuxième joint hermétique.

5. Boîtier hermétique selon l'une des revendications précédentes, dans lequel la partie externe dudit ou de chaque cadre métallique présente, par rapport à la partie interne correspondante, une surépaisseur sur sa face opposée audit deuxième joint hermétique.

6. Boîtier hermétique selon l'une des revendications précédentes, présentant une épaisseur inférieure ou égale à 1 mm, et de préférence inférieure ou égale à 500 µm.

7. Boîtier hermétique selon l'une des revendications précédentes, comprenant au moins un composant (41, 93) monté sur ledit premier élément en forme de feuille, directement ou par l'intermédiaire d'une couche diélectrique, et contenu dans un espace délimité par ledit premier élément, ledit deuxième élément et ledit ou lesdits cadres métalliques.

8. Boîtier hermétique selon la revendication précédente, comprenant au moins une piste conductrice (53) agencée sur ledit premier élément en forme de feuille, ladite ou chaque piste conductrice s'étendant sur les deux faces et sur la tranche dudit premier élément et étant recouverte d'un matériau isolant (54, 55) sauf au niveau de ses extrémités, formant plages de connexion.

9. Boîtier hermétique selon l'une des revendications 2 à 8, dans lequel la partie interne de la feuille métallique dite supérieure (15, 65) est concave, en particulier pour définir une cavité.

10. Boîtier hermétique selon la revendication précédente, dans lequel la partie externe de la feuille métallique supérieure (15, 65) est également concave.

11. Boîtier hermétique selon l'une des revendications précédentes, dans lequel le premier élément en forme de feuille (11, 61) présente une forme en U, en particulier pour définir une cavité.

12. Boîtier hermétique selon l'une des revendications précédentes, comprenant :
- un premier élément en forme de feuille (11, 61) réalisé en céramique ;
- un cadre additionnel (132, 142) également réalisé en céramique ; et
- une ou plusieurs électrodes (131, 141) métalliques, la ou chaque électrode métallique (131, 141) présentant une portion (1313) prise en sandwich entre le premier élément en forme de feuille (11, 61) et le cadre additionnel en céramique (132, 142), une première extrémité (1311, 1411) accessible sur une face supérieure (52) du premier élément en forme de feuille (11, 61) et une deuxième extrémité (1312, 1412) accessible soit sur une face inférieure (56) du premier élément en forme de feuille (11, 61) soit sur une face inférieure (1422) du cadre additionnel en céramique (142).

13. Boîtier selon la revendication précédente, dans lequel il est prévu un joint hermétique entre la partie interne (14, 64) du premier cadre métallique (12, 62) et le cadre additionnel en céramique (132, 142).

14. Boîtier hermétique selon l'une des revendications 1 à 11, comprenant :
- un premier élément en forme de feuille (11, 61) réalisé en métal ;
- un premier cadre additionnel (152), réalisé en céramique ;
- un deuxième cadre additionnel (153), également réalisé en céramique ;
- une ou plusieurs électrodes (151) métalliques, la ou chaque électrode métallique (151) présentant une portion (1513) prise en sandwich entre le premier cadre additionnel en céramique (152) et le deuxième cadre additionnel en céramique (153), une première extrémité (1511) accessible sur une face supérieure (1521) du premier cadre additionnel en céramique (152) et une deuxième extrémité (1512) accessible sur une face inférieure (1532) du deuxième cadre additionnel en céramique (153).

15. Boîtier hermétique selon la revendication précédente, dans lequel il est prévu un joint hermétique entre la partie interne du premier cadre métallique (12, 62) et le deuxième cadre additionnel en céramique (153).

16. Boîtier hermétique selon l'une des revendications 14 ou 15, dans lequel il est prévu un joint hermétique entre la face supérieure (52) du premier élément en forme de feuille (11,61) et la face inférieure (1522) du premier cadre additionnel en céramique (152).

17. Boîtier hermétique selon l'une des revendications précédentes, présentant la forme d'une croix (122) de sorte qu'il puisse être courbé selon plusieurs axes, en particulier non parallèles entre eux.

18. Dispositif comprenant plusieurs boîtiers selon l'une des revendications précédentes, **caractérisé en ce que** lesdits boîtiers sont reliés mécaniquement entre eux par un support commun flexible (124), par exemple réalisé en polymère, pour améliorer la flexibilité de l'ensemble ainsi formé par lesdits boîtiers.

19. Dispositif selon la revendication précédente, dans lequel le support commun flexible (124) est collé aux boîtiers ou surmoulé, partiellement ou complètement, sur lesdits boîtiers.

20. Dispositif selon l'une des revendications 18 ou 19, dans lequel le support commun flexible (124) comprend des pistes ou fils métalliques pour relier électriquement des boîtiers entre eux.

21. Dispositif selon l'une des revendications 18 à 20, dans lequel le support commun flexible (124) comprend un ou plusieurs composants électroniques, par exemple une antenne.

22. Dispositif selon l'une des revendications 18 à 21, dont les boîtiers sont agencés pour conférer une forme de croix audit dispositif, de sorte que ce dernier puisse être courbé selon plusieurs axes, en particulier non parallèles entre eux.

23. Procédé d'encapsulation d'un dispositif dans un boîtier hermétique selon l'une des revendications 1 à 17, comprenant les étapes consistant à :
- monter au moins un composant (41, 93) dudit dispositif à encapsuler sur le premier élément en forme de feuille (11, 61), réalisé en au moins un matériau choisi parmi un métal, une céramique et un verre ;
- superposer audit premier élément en forme de feuille le premier cadre métallique (12, 62) comprenant ladite partie interne (14, 64) positionnée en partie ou en totalité à l'intérieur du périmètre dudit premier élément et ladite partie externe (13, 63) positionnée en totalité à l'extérieur dudit périmètre dudit premier élément ;
- former le premier joint hermétique (31) entre ledit premier élément et ladite partie interne dudit premier cadre métallique ;
- superposer audit premier cadre métallique ledit deuxième élément (15, 16 ; 65) de dimensions et forme adaptés pour recouvrir ledit premier élément, également réalisé en au moins un matériau choisi parmi un métal, une céramique et un verre ; et
- former le deuxième joint hermétique entre ledit deuxième élément et ladite partie externe dudit premier cadre métallique.

24. Procédé selon la revendication précédente, dans lequel l'étape de formation dudit premier joint hermétique est réalisée par une technique choisie parmi : le brasage, le soudage par diffusion à l'état solide et, lorsque ledit deuxième élément est au moins en partie en céramique, le cofrittage.

25. Procédé selon l'une des revendications 23 ou 24, dans lequel l'étape de formation dudit deuxième joint hermétique est réalisée par soudage.

26. Procédé selon l'une des revendications 23 à 25, dans lequel ledit deuxième élément comprend :
- une feuille dite supérieure (15), réalisée en au moins un matériau choisi parmi un métal, une céramique et un verre ;
- un deuxième cadre métallique (16) interposé entre ladite feuille supérieure et ledit premier cadre métallique et comprenant une partie interne (18) positionnée en partie ou en totalité à l'intérieur du périmètre de ladite feuille supérieure et une partie externe (17) positionnée en totalité à l'extérieur dudit périmètre de ladite feuille supérieure ;
le procédé comprenant également une étape de formation d'un troisième joint hermétique (32) entre ladite feuille supérieure et ladite partie interne dudit deuxième cadre métallique, ladite étape étant réalisée avant la formation dudit deuxième joint hermétique entre les parties externes desdits premier et deuxième cadre métallique.

27. Procédé selon l'une des revendications 23 à 26, dans lequel ledit deuxième élément comprend une feuille dite supérieure (65) en métal, et dans lequel ledit deuxième joint hermétique est formé directement entre ladite feuille supérieure et la partie externe dudit premier cadre métallique.

## Patentansprüche

1. Ultradünnes hermetisches Gehäuse, das eine Dicke von unter oder gleich 3 mm aufweist, wobei das ultradünne hermetische Gehäuse umfasst:
- ein erstes Element in Folienform (11, 61), hergestellt aus mindestens einem Material, ausgewählt aus einem Metall, einer Keramik und einem Glas,
- ein zweites Element (15, 16; 65) in Abmessungen und in einer Form, die geeignet sind, um das erste Element zu bedecken, das ebenfalls aus mindestens einem Material hergestellt ist, das aus einem Metall, einer Keramik und einem Glas ausgewählt ist,
- einen ersten Metallrahmen (12, 62) zwischen dem ersten Element und dem zweiten Element, umfassend einen inneren Teil (14, 64), der teilweise oder ganz im Innern des Umfangs des ersten Elements positioniert ist, und einen äußeren Teil (13, 63), der ganz außerhalb des Umfangs des ersten Elements positioniert ist,
- eine erste hermetische Naht (31) zwischen dem ersten Element und dem inneren Teil des ersten Metallrahmens, und
- eine zweite hermetische Naht zwischen dem zweiten Element und dem äußeren Teil des ersten Metallrahmens.

2. Hermetisches Gehäuse nach Anspruch 1, wobei das zweite Element umfasst:
- eine obere Folie (15), die aus mindestens einem Material hergestellt ist, das aus einem Metall, einer Keramik und einem Glas ausgewählt ist,
- einen zweiten Metallrahmen (16) zwischen der obere Folie und dem ersten Metallrahmen, umfassend einen inneren Teil (18), der teilweise oder ganz im Innern des Umfangs der oberen Folie positioniert ist, und einen äußeren Teil (17), der ganz außerhalb des Umfangs der oberen Folie positioniert ist, und
- eine dritte hermetische Naht (32) zwischen der oberen Folie und dem inneren Teil des zweiten Metallrahmens,
wobei die zweite hermetische Naht zwischen den äußeren Teilen des ersten und zweiten Metallrahmens ist.

3. Hermetisches Gehäuse nach vorangehendem Anspruch, wobei die dritte hermetische Naht mit einer Technik hergestellt ist, die aus dem Löten, dem Schweißen durch Diffusion in festem Zustand und, wenn die obere Folie mindestens teilweise aus Keramik ist, dem Co-Sintern ausgewählt ist.

4. Hermetisches Gehäuse nach Anspruch 1, wobei das zweite Element eine obere Folie (65) aus Metall umfasst, die direkt am äußeren Teil des ersten Metallrahmens mittels der zweiten hermetischen Naht befestigt ist.

5. Hermetisches Gehäuse nach einem der vorangehenden Ansprüche, wobei der äußere Teil des oder jedes Metallrahmens in Bezug zum entsprechenden inneren Teil eine Dickenzugabe auf seiner der zweiten hermetischen Naht gegenüberliegenden Seite aufweist.

6. Hermetisches Gehäuse nach einem der vorangehenden Ansprüche, das eine Dicke von unter oder gleich 1 mm und vorzugsweise von unter oder gleich 500 µm aufweist.

7. Hermetisches Gehäuse nach einem der vorangehenden Ansprüche, umfassend mindestens ein Bauteil (41, 93), das direkt oder mittels einer dielektrischen Schicht auf dem ersten Element in Folienform angebracht und in einem Raum enthalten ist, der von dem ersten Element, dem zweiten Element und dem oder den Metallrahmen begrenzt ist.

8. Hermetisches Gehäuse nach vorangehendem Anspruch, umfassend mindestens eine Leitungsbahn (53), die auf dem ersten Element in Folienform ausgebildet ist, wobei sich die oder jede Leitungsbahn über die zwei Seiten und über die Kante des ersten Elements erstreckt und mit einem Isolationsmaterial (54, 55) bedeckt ist, außer an ihren Enden, die Verbindungsbereiche bilden.

9. Hermetisches Gehäuse nach einem der Ansprüche 2 bis 8, wobei der innere Teil der oberen Metallfolie (15, 65) konkav ist, insbesondere, um einen Hohlraum zu definieren.

10. Hermetisches Gehäuse nach vorangehendem Anspruch, wobei der äußere Teil der oberen Metallfolie (15, 65) ebenfalls konkav ist.

11. Hermetisches Gehäuse nach einem der vorangehenden Ansprüche, wobei das erste Element in Folienform (11, 61) eine U-Form aufweist, insbesondere, um einen Hohlraum zu definieren.

12. Hermetisches Gehäuse nach einem der vorangehenden Ansprüche, umfassend:
- ein erstes Element in Folienform (11, 61), das aus Keramik hergestellt ist,
- einen zusätzlichen Rahmen (132, 142), der ebenfalls aus Keramik hergestellt ist, und
- eine oder mehrere Metallelektroden (131, 141), wobei die oder jede Metallelektrode (131, 141) eine Abschnitt (1313) aufweist, der sandwichartig zwischen dem ersten Element in Folienform (11, 61) und dem zusätzlichen Rahmen aus Keramik (132, 142) eingeschlossen ist, ein erstes Ende (1311, 1411), das über eine Oberseite (52) des ersten Elements in Folienform (11, 61) erreichbar ist und ein zweites Ende (1312, 1412), das entweder über eine Unterseite (56) des ersten Elements in Folienform (11, 61) oder über eine Unterseite (1422) des zusätzlichen Rahmens aus Keramik (142) erreichbar ist.

13. Gehäuse nach vorangehendem Anspruch, wobei eine hermetische Naht zwischen dem inneren Teil (14, 64) des ersten Metallrahmens (12, 62) und dem zusätzlichen Rahmen aus Keramik (132, 142) vorgesehen ist.

14. Hermetisches Gehäuse nach einem der Ansprüche 1 bis 11, umfassend:
- ein erstes Element in Folienform (11, 61), das aus Metall hergestellt ist,
- einen ersten zusätzlichen Rahmen (152), der aus Keramik hergestellt ist,
- einen zweiten zusätzlichen Rahmen (153), der ebenfalls aus Keramik hergestellt ist,
- eine oder mehrere Metallelektroden (151), wobei die oder jede Metallelektrode (151) eine Abschnitt (1513) aufweist, der sandwichartig zwischen dem ersten zusätzlichen Rahmen aus Keramik (152) und dem zweiten zusätzlichen Rahmen aus Keramik (153) eingeschlossen ist, ein erstes Ende (1511), das über eine Oberseite (1521) des ersten zusätzlichen Rahmens aus Keramik (152) erreichbar ist und ein zweites Ende (1512), das über eine Unterseite (1532) des zweiten zusätzlichen Rahmens aus Keramik (153) erreichbar ist.

15. Hermetisches Gehäuse nach vorangehendem Anspruch, wobei eine hermetische Naht zwischen dem inneren Teil des ersten Metallrahmens (12, 62) und dem zweiten zusätzlichen Rahmen aus Keramik (153) vorgesehen ist.

16. Hermetisches Gehäuse nach einem der Ansprüche 14 oder 15, wobei eine hermetische Naht zwischen der Oberseite (52) des ersten Elements in Folienform (11, 61) und der Unterseite (1522) des ersten zusätzlichen Rahmens aus Keramik (152) vorgesehen ist.

17. Hermetisches Gehäuse nach einem der vorangehenden Ansprüche, aufweisend die Form eines Kreuzes (122), so dass es gemäß mehreren Achsen gekrümmt sein kann, die insbesondere nicht parallel zueinander sind.

18. Vorrichtung, umfassend mehrere Gehäuse nach einem der vorangehenden Ansprüche, **dadurch gekennzeichnet, dass** die Gehäuse mittels einer gemeinsamen flexiblen Basis (124), die beispielsweise aus Polymer hergestellt ist, um die Flexibilität der derart von den Gehäusen gebildeten Einheit zu verbessern, mechanisch miteinander verbunden sind.

19. Vorrichtung nach vorangehendem Anspruch, wobei die gemeinsame flexible Basis (124) mit den Gehäusen verklebt ist oder teilweise oder vollständig auf die Gehäuse aufgeformt ist.

20. Vorrichtung nach einem der Ansprüche 18 oder 19, wobei die gemeinsame flexible Basis (124) Bahnen oder Metalldrähte umfasst, um die Gehäuse miteinander elektrisch zu verbinden.

21. Vorrichtung nach einem der Ansprüche 18 bis 20, wobei die gemeinsame flexible Basis (124) ein oder mehrere elektronische Bauteile, beispielsweise eine Antenne, umfasst.

22. Vorrichtung nach einem der Ansprüche 18 bis 21, wobei die Gehäuse ausgebildet sind, um der Vorrichtung eine Kreuzform zu verleihen, so dass sie gemäß mehreren Achsen gekrümmt sein kann, die insbesondere nicht parallel zueinander sind.

23. Verfahren zum Einkapseln einer Vorrichtung in ein hermetisches Gehäuse nach einem der Ansprüche 1 bis 17, das die folgenden Schritte umfasst:
- Anbringen mindestens eines Bauteils (41, 93) der einzukapselnden Vorrichtung auf das erste Element in Folienform (11, 61), das aus mindestens einem Material hergestellt ist, das aus einem Metall, einer Keramik und einem Glas ausgewählt ist,
- Setzen auf das erste Element in Folienform des ersten Metallrahmens (12, 62), umfassend den inneren Teil (14, 64), der teilweise oder ganz im Innern des Umfangs des ersten Elements positioniert ist, und den äußeren Teil (13, 63), der ganz außerhalb des Umfangs des ersten Elements positioniert ist,
- Bilden der ersten hermetischen Naht (31) zwischen dem ersten Element und dem inneren Teil des ersten Metallrahmens,
- Setzen auf den ersten Metallrahmen das zweite Element (15, 16; 65) in Abmessungen und in einer Form, die geeignet sind, um das erste Element zu bedecken, das ebenfalls aus mindestens einem Material hergestellt ist, das aus einem Metall, einer Keramik und einem Glas ausgewählt ist, und
- Bilden der zweiten hermetischen Naht zwischen dem zweiten Element und dem äußeren Teil des ersten Metallrahmens.

24. Verfahren nach vorangehendem Anspruch, wobei der Schritt der Bildung der ersten hermetischen Naht mit einer Technik durchgeführt wird, die aus dem Löten, dem Schweißen durch Diffusion in festem Zustand und, wenn das zweite Element mindestens teilweise aus Keramik ist, dem Co-Sintern ausgewählt ist.

25. Verfahren nach einem der Ansprüche 23 oder 24, wobei der Schritt der Bildung der zweiten hermetischen Naht durch Schweißen durchgeführt wird.

26. Verfahren nach einem der Ansprüche 23 bis 25, wobei das zweite Element umfasst:
- eine obere Folie (15), die aus mindestens einem Material hergestellt ist, das aus einem Metall, einer Keramik und einem Glas ausgewählt ist,
- einen zweiten Metallrahmen (16) zwischen der oberen Folie und dem ersten Metallrahmen, umfassend einen inneren Teil (18), der teilweise oder ganz im Innern des Umfangs der oberen Folie positioniert ist, und einen äußeren Teil (17), der ganz außerhalb des Umfangs der oberen Folie positioniert ist,
wobei das Verfahren ebenfalls einen Schritt der Bildung einer dritten hermetischen Naht (32) zwischen der oberen Folie und dem inneren Teil des zweiten Metallrahmens umfasst, wobei der Schritt vor der Bildung der zweiten hermetischen Naht zwischen den äußeren Teilen des ersten und zweiten Metallrahmens durchgeführt wird.

27. Verfahren nach einem der Ansprüche 23 bis 26, wobei das zweite Element eine obere Folie (65) aus Metall umfasst und wobei die zweite hermetische Naht direkt zwischen der oberen Folie und dem äußeren Teil des ersten Metallrahmens gebildet wird.

## Claims

1. An ultrathin hermetic housing exhibiting a thickness of less than or equal to 3 mm, the ultrathin hermetic housing comprising:
- a first element in the form of a sheet (11, 61), made of at least one material chosen from a metal, a ceramic and a glass;
- a second element (15, 16; 65) having dimensions and a shape suitable for covering said first element, also made of at least one material chosen from a metal, a ceramic and a glass;
- a first metal surround (12, 62) interposed between said first element and said second element, comprising an internal part (14, 64) positioned partially or completely inside the perimeter of said first element and an external part (13, 63) positioned completely outside said perimeter of said first element;
- a first hermetic seal (31) between said first element and said internal part of said first metal surround; and
- a second hermetic seal between said second element and said external part of said first metal surround.

2. The hermetic housing according to claim 1, wherein said second element comprises:
- an "upper" sheet (15), made of at least one material chosen from a metal, a ceramic and a glass;
- a second metal surround (16) interposed between said upper sheet and said first metal surround, comprising an internal part (18) positioned partially or completely inside the perimeter of said upper sheet and an external part (17) positioned completely outside said perimeter of said upper sheet; and
- a third hermetic seal (32) between said upper sheet and said internal part of said second metal surround;
said second hermetic seal being between the external parts of said first and second metal surrounds.

3. The hermetic housing according to the preceding claim, wherein said third hermetic seal is produced by a technique chosen from: soldering, solid state diffusion welding and, when said upper sheet is at least partially made of ceramic, cosintering.

4. The hermetic housing according to claim 1, wherein said second element comprises an "upper" sheet (65) made of metal, directly attached to the external part of said first metal surround via said second hermetic seal.

5. The hermetic housing according to one of the preceding claims, wherein the external part of said or of each metal surround exhibits, with respect to the corresponding internal part, an excess thickness over its face opposite said second hermetic seal.

6. The hermetic housing according to one of the preceding claims, exhibiting a thickness of less than or equal to 1 mm, and preferably of less than or equal to 500 µm.

7. The hermetic housing according to one of the preceding claims, comprising at least one component (41, 93) mounted on said first element in the form of a sheet, directly or via a dielectric layer, and contained in a space delimited by said first element, said second element and said metal surround(s).

8. The hermetic housing according to the preceding claim, comprising at least one conductive track (53) arranged on said first element in the form of a sheet, said or each conductive track extending over both faces and over the side face of said first element and being covered with an insulating material (54, 55), except at its ends, forming lands.

9. The hermetic housing according to one of claims 2 to 8, wherein the internal part of the "upper" metal sheet (15, 65) is concave, in particular in order to define a cavity.

10. The hermetic housing according to the preceding claim, wherein the external part of the upper metal sheet (15, 65) is also concave.

11. The hermetic housing according to one of the preceding claims, wherein the first element in the form of a sheet (11, 61) exhibits a U shape, in particular in order to define a cavity.

12. The hermetic housing according to one of the preceding claims, comprising:
- a first element in the form of a sheet (11, 61) made of a ceramic;
- an additional surround (132, 142) also made of ceramic; and
- one or more metal electrodes (131, 141), the or each metal electrode (131, 141) exhibiting a portion (1313) sandwiched between the first element in the form of a sheet (11, 61) and the additional surround made of ceramic (132, 142), a first end (1311, 1411) accessible on an upper face (52) of the first element in the form of a sheet (11, 61) and a second end (1312, 1412) accessible either on a lower face (56) of the first element in the form of a sheet (11, 61) or on a lower face (1422) of the additional surround made of ceramic (142).

13. The housing according to the preceding claim, wherein a hermetic seal is provided between the internal part (14, 64) of the first metal surround (12, 62) and the additional surround made of ceramic (132, 142).

14. The hermetic housing according to one of claims 1 to 11, comprising:
- a first element in the form of a sheet (11, 61) made of metal;
- a first additional surround (152) made of ceramic;
- a second additional surround (153), also made of ceramic;
- one or more metal electrodes (151), the or each metal electrode (151) exhibiting a portion (1513) sandwiched between the first additional surround made of ceramic (152) and the second additional surround made of ceramic (153), a first end (1511) accessible on an upper face (1521) of the first additional surround made of ceramic (152) and a second end (1512) accessible on a lower face (1532) of the second additional surround made of ceramic (153).

15. The hermetic housing according to the preceding claim, wherein a hermetic seal is provided between the internal part of the first metal surround (12, 62) and the second additional surround made of ceramic (153).

16. The hermetic housing according to one of claims 14 or 15, wherein a hermetic seal is provided between the upper face (52) of the first element in the form of a sheet (11, 61) and the lower face (1522) of the first additional surround made of ceramic (152).

17. The hermetic housing according to one of the preceding claims, exhibiting the shape of a cross (122) so that it can be curved along several axes, in particular axes which are nonparallel with one another.

18. A device comprising several housings according to one of the preceding claims, **characterized in that** said housings are connected mechanically to one another by a flexible common support (124), for example made of polymer, in order to improve the flexibility of the assembly thus formed by said housings.

19. The device according to the preceding claim, wherein the flexible common support (124) is adhesively bonded to the housings or overmolded, partially or completely, over said housings.

20. The device according to one of claims 18 or 19, wherein the flexible common support (124) comprises metal tracks or wires in order to electrically connect housings to one another.

21. The device according to one of claims 18 to 20, wherein the flexible common support (124) comprises one or more electronic components, for example an antenna.

22. The device according to one of claims 18 to 21, the housings of which are arranged in order to confer a cross shape on said device, so that the latter can be curved along several axes, in particular axes which are nonparallel with one another.

23. A process for the encapsulation of a device in a hermetic housing according to one of claims 1 to 17, comprising the stages consisting in:
- mounting at least one component (41, 93) of said device to be encapsulated on the first element in the form of a sheet (11, 61), made of at least one material chosen from a metal, a ceramic and a glass;
- superimposing, on said first element in the form of a sheet, the first metal surround (12, 62) comprising said internal part (14, 64) positioned partially or completely inside the perimeter of said first element and said external part (13, 63) positioned completely outside said perimeter of said first element;
- forming the first hermetic seal (31) between said first element and said internal part of said first metal surround;
- superimposing, on said first metal surround, said second element (15, 16; 65) with dimensions and a shape suitable for covering said first element, also made of at least one material chosen from a metal, a ceramic and a glass; and
- forming the second hermetic seal between said second element and said external part of said first metal surround.

24. The process according to the preceding claim, wherein the stage of formation of said first hermetic seal is carried out by a technique chosen from: soldering, solid state diffusion welding and, when said second element is at least partially made of ceramic, cosintering.

25. The process according to one of claims 23 or 24, wherein the stage of formation of said second hermetic seal is carried out by welding.

26. The process according to one of claims 23 to 25, wherein said second element comprises:
- an "upper" sheet (15), made of at least one material chosen from a metal, a ceramic and a glass;
- a second metal surround (16) interposed between said upper sheet and said first metal surround and comprising an internal part (18) positioned partially or completely inside the perimeter of said upper sheet and an external part (17) positioned completely outside said perimeter of said upper sheet;
the process also comprising a stage of formation of a third hermetic seal (32) between said upper sheet and said internal part of said second metal surround, said stage being carried out before the formation of said second hermetic seal between the external parts of said first and second metal surrounds.

27. The process according to one of claims 23 to 26, wherein said second element comprises an "upper" sheet (65) made of metal and wherein said second hermetic seal is formed directly between said upper sheet and the external part of said first metal surround.
